# EUROPEAN PATENT APPLICATION

(11) **EP 4 509 033 A1**
(43) Date of publication of application: **19.02.2025**
(21) Application number: 24194690.4
(22) Date of filing: 15.08.2024
(51) Int. Cl.: A61B 3/00, A61B 3/032, A61B 3/08, A61B 3/09, A61B 3/103, A61B 3/107

(54) **OPHTHALMOLOGIC APPARATUS AND METHOD FOR EXAMINING SUBJECT EYES**

(30) Priority: 16.08.2023 JP 2023132661
(71) Applicant: OSAKA UNIVERSITY, Suita-shi Osaka 565-0871 (JP); TOPCON CORPORATION, Tokyo 174-8580 (JP)
(72) Inventor: FUJIKADO, Takashi, Osaka, 565-0871 (JP); TATARA, Yoko, Tokyo, 174-8580 (JP); YUKIMORI, Takafumi, Tokyo, 174-8580 (JP); SAIKA, Makoto, Tokyo, 174-8580 (JP); NORO, Ryoka, Tokyo, 174-8580 (JP); SAKAIHARA, Manabu, Tokyo, 174-8580 (JP)
(74) Representative: Louis Pöhlau Lohrentz

(57) **Abstract**

An ophthalmologic apparatus (100) includes a visual target presenting portion (4) that presents fixation targets (500L, 500R) to subject eyes (E); an objective measurement optical system (6, 7) that objectively measures eye characteristics of the subject eyes (E); and a controller (140). Each of the fixation targets (500L, 500R) includes fusion targets (502L, 502R) depicted in a manner that allows binocular fusion while the subject eyes (E) respectively view the fixation targets (500L, 500R). The visual target presenting portion (4) presents the fixation targets (500L, 500R) while changing an examination distance from the subject eyes (E) to the fixation target (500L, 500R) when an anisometropia is induced. In the anisometropia, one subject eye (E) is in a fully corrected condition and the other subject eye (E) is corrected by a predetermined degree from the fully corrected condition. The controller (140) controls the objective measurement optical system (6, 7) to measure the eye characteristics while the subject eyes (E) binocularly view the fixation targets (500L, 500R) and acquire refractive values of the subject eyes (E) as objective measurement information.

## Description

### FIELD OF THE INVENTION

The present invention relates to an ophthalmologic apparatus and a method for examining subject eyes.

### BACKGROUND

A treatment method called monovision has been conventionally used to allow eyes to see objects from a distance to close range when viewed binocularly with both eyes by correcting each vision of the subject eyes to enable one of the subject eyes to see objects from a distance and the other to see objects at close distance. Monovision prescription may bring about anisometropia, in which the refractive value (visual acuity) of one eye greatly differs from that of the other eye. Tolerance for anisometropia varies from person to person. JP2006-122661A discloses a method for estimating the monovision prescription applicability to a person (whether it is easy to apply monovision to the person or not) based on his/her ocular dominance.

JP2013-195931A discloses an ophthalmologic apparatus that allows the examinee to experience binocularly viewing with both eyes under a monovision prescription to check for any discomfort.

### SUMMARY

Applying the monovision prescription can be deemed suitable if the targeted subject eye can establish visual fixation while changing the distance from the subject eyes to the fixation target under an anisometropia condition. Accordingly, the applicability of the monovision prescription can be precisely assessed by determining the fixation eye depending on the examination distance under the anisometropia condition and by determining whether or not the fixation eye switches between the far-point vision and the near-point vision. Prior technologies determine the applicability of the monovision prescription based on the ocular dominance and the check for of the discomfort when binocularly viewed with both eyes under the monovision prescription, but do not determine the fixation eye by varying examination distances under the anisometropia condition. The term "fixation eye" refers to one of the subject person's eyes, either the left or right, that the subject person uses primarily to see an object mainly and to focus the object substantially at the fovea centralis.

The present invention has been made by considering the above problem. An object of the present invention aims to provide an ophthalmologic apparatus and a method for examining subject eyes that can determine the fixation eye at each examination distance under an anisometropia condition.

According to a first aspect of the present invention, an ophthalmologic apparatus includes a visual target presenting portion that is configured to present fixation targets to left and right subject eyes, respectively; an objective measurement optical system that is configured to objectively measure eye characteristics of the left and right subject eyes, respectively; and a controller that is configured to control the objective measurement optical system. Each of the fixation targets includes a fusion target that is depicted in a manner that allows binocular fusion the left and right subject eyes respectively view the fixation targets. The visual target presenting portion is configured to present the fixation targets while changing the examination distance from the left and right subject eyes to the fixation targets in a predetermined range of the examination distance when an anisometropia is induced. In the anisometropia, one of the left and right subject eyes is maintained in a fully corrected condition and another of the left and right subject eyes is corrected by a predetermined degree from the fully corrected condition. The controller is configured to control the objective measurement optical system to measure the eye characteristics while the left and right subject eyes binocularly view the fixation targets and acquire refractive values of the left and right subject eyes as objective measurement information.

According to a second aspect of the present invention, a method for examining subject eyes by an ophthalmologic apparatus is provided. The ophthalmologic apparatus includes a visual target presenting portion that is configured to present fixation targets to left and right subject eyes, respectively; an objective measurement optical system that is configured to objectively measure eye characteristics of the left and right subject eyes, respectively; and a controller that is configured to control the objective measurement optical system. The method includes a first step wherein under a condition of an anisometropia in which one of the left and right subject eyes is maintained in a fully corrected condition and another of the left and right subject eyes is corrected by a predetermined power from the fully corrected condition, the visual target presenting portion presents the fixation targets and changes an examination from the left and right subject eyes to the fixation target in a predetermined range of the examination distance, and the controller controls the objective measurement optical system to measure the eye characteristics while the left and right subject eyes binocularly view the fixation targets and acquire refractive values of the left and right subject eyes as objective measurement information; and a second step wherein after acquiring the objective measurement information in the first step, under a condition of an anisometropia in which the another of the left and right subject eyes is maintained in the fully corrected condition and in which the one of the left and right subject eyes is corrected by a predetermined power from the fully corrected condition, the visual target presenting portion presents the fixation targets and changes the examination distance in the predetermined range of the examination distance, and the controller controls the objective measurement optical system to measure the eye characteristics while both of the left and right subject eyes binocularly view the fixation targets and acquire refractive values of the left and right subject eyes as the objective measurement information

Thus, it is possible to identify the fixation eye at every test distance under the anisometropia condition by the ophthalmologic apparatus and the method for testing subject eyes of the present invention.

### BRIEF DESCRIPTION OF DRAWINGS

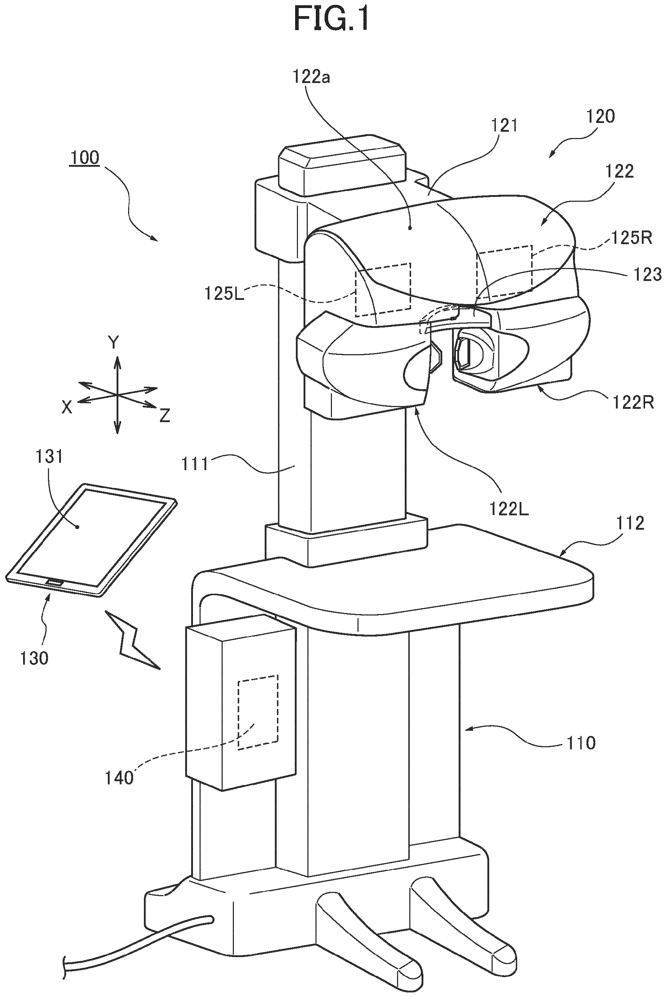

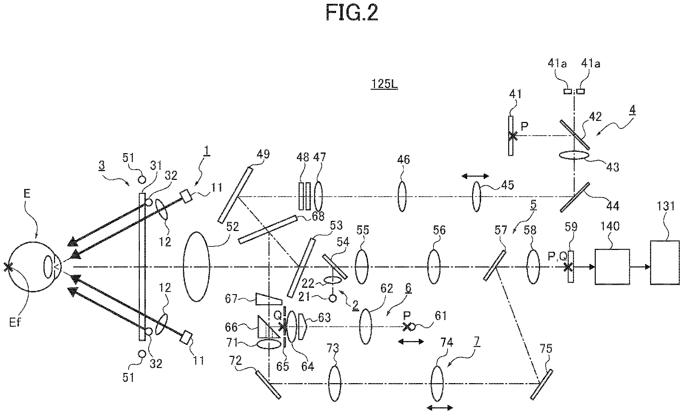

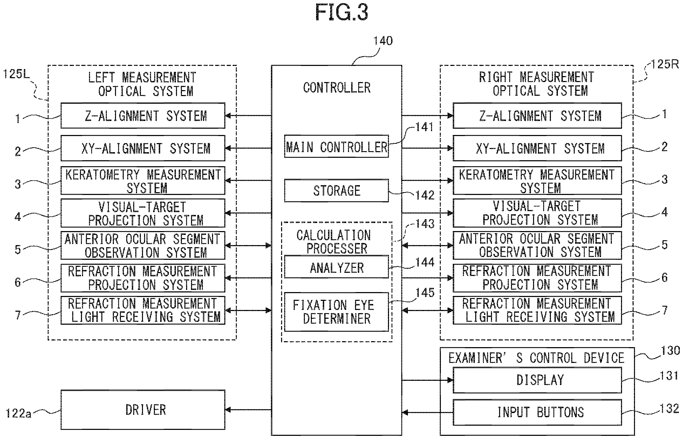

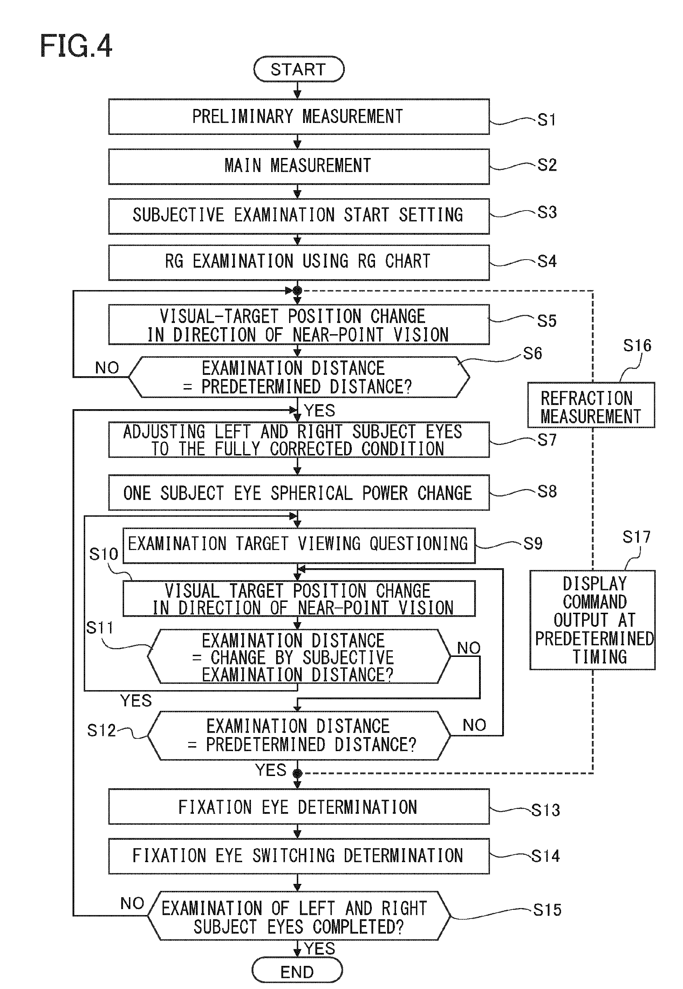

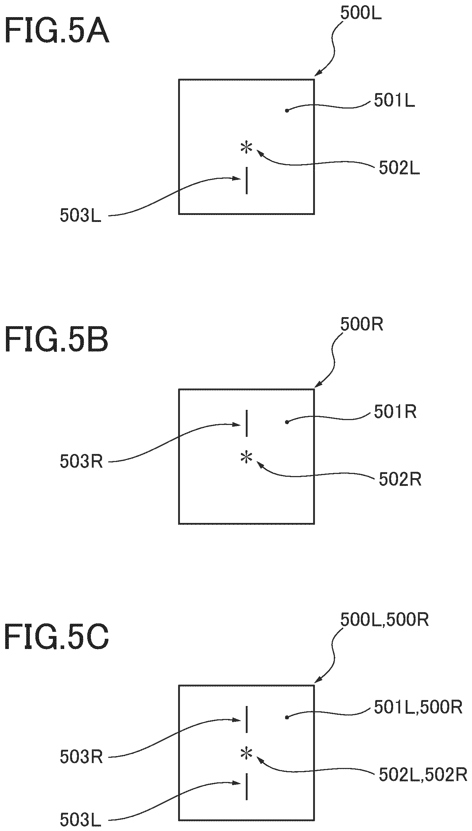

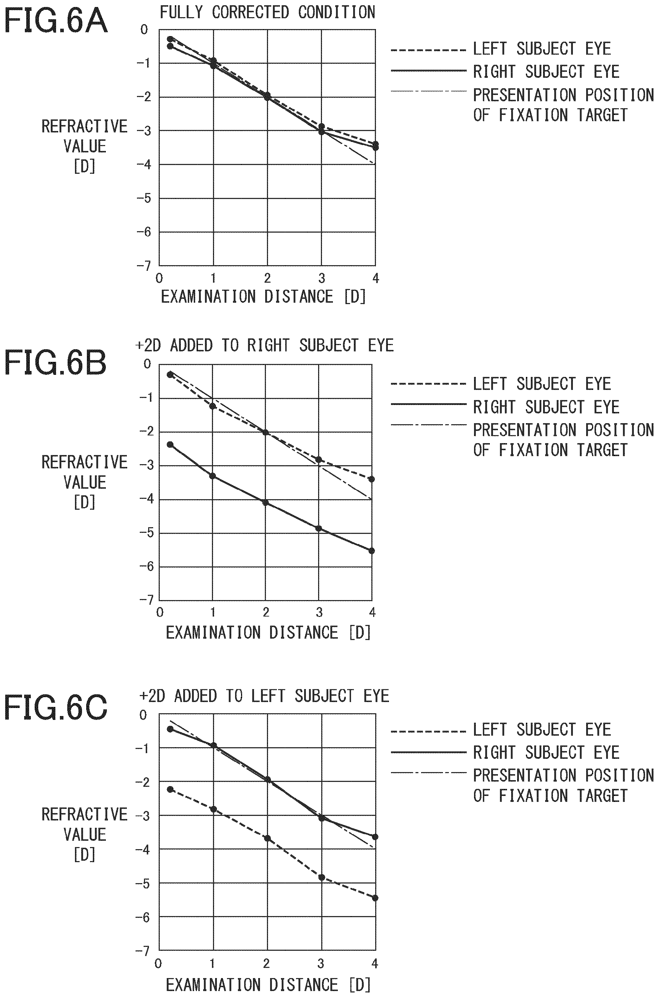

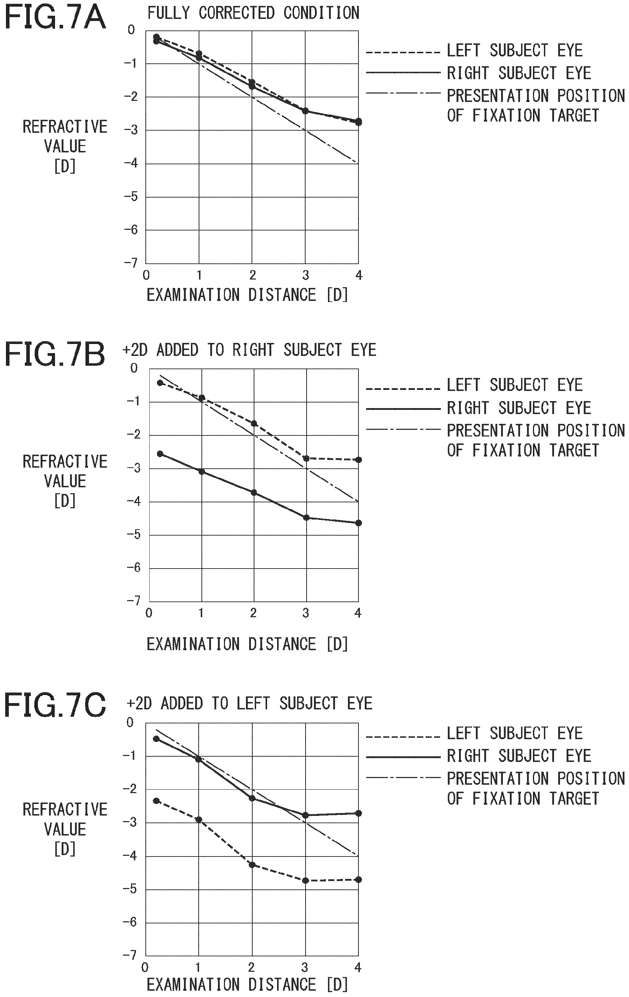

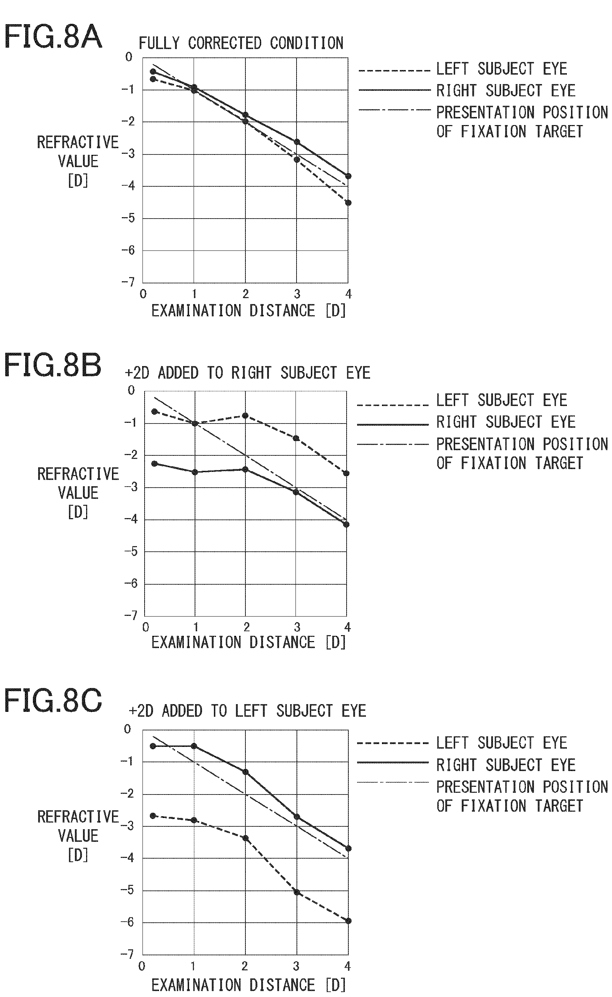

FIG. 1 is an external perspective view showing an ophthalmologic apparatus according to a first embodiment. FIG. 2 is a schematic view showing an exemplary configuration of a left measurement optical system of the ophthalmologic apparatus according to the first embodiment. FIG. 3 is a block diagram showing a control configuration of the ophthalmologic apparatus according to the first embodiment. FIG. 4 is a flowchart showing a flow of processing steps of a method for examining subject eyes with the ophthalmologic apparatus according to the first embodiment. FIG. 5A is an explanatory view showing a left fixation target. FIG. 5B is an explanatory view showing a right fixation target. FIG. 5C is an explanatory view showing how the left and right fixation targets are seen when viewed by the left and right subject eyes, respectively. FIG. 6A is a graph showing the examination distance and the refractive value for a first examinee when both eyes are fully corrected. FIG. 6B is a graph showing the examination distance and the refractive value when +2D is added to the right eye of the first examinee. FIG. 6C is a graph showing the examination distance and the refractive value when +2D is added to the left eye of the first examinee. FIG. 7A is a graph showing the examination distance and the refractive value for a second examinee when both eyes are fully corrected. FIG. 7B is a graph showing the examination distance and the refractive value when +2D is added to the right eye of the second examinee. FIG. 7C is a graph showing the examination distance and the refractive value when +2D is added to the left eye of the second examinee. FIG. 8A is a graph showing the examination distance and the refractive value for a third examinee when both eyes are fully corrected. FIG. 8B is a graph showing the examination distance and the refractive value when +2D is added to the right eye of the third examinee. FIG. 8C is a graph showing the examination distance and the refractive value when +2D is added to the left eye of the third examinee.

### DETAILED DESCRIPTION

For the use of plural and/or singular terms herein, those having skill in the art can translate from the plural to the singular and/or from the singular to the plural as is appropriate to the context and/or application. The various singular/plural permutations may be expressly set forth herein for the sake of clarity.

An embodiment of an ophthalmologic apparatus and a method for examining subject eyes according to the present invention will be described based on a first embodiment illustrated in the drawings.

An ophthalmologic apparatus 100 of the first embodiment is a both-eye open type apparatus capable of simultaneously measuring the eye characteristics of both eyes at the same time with an examinee opening both eyes. The ophthalmologic apparatus 100 may also individually measure the eye characteristics of each eye at a time by shielding one of the eyes or turning off a fixed target for the one of the eyes. The ophthalmologic apparatus 100 of the first embodiment is an objective measurement apparatus with subjective measurement functions and the objective measurement apparatus includes a visual target presenting function, a phoropter function, and an automatic refraction and keratometry measurement function. This ophthalmologic apparatus 100 allows the examiner to objectively and subjectively measure the eye characteristics of subject eyes E with the objective and subjective examinations.

The objective examinations the ophthalmologic apparatus 100 of the first embodiment can execute include the measurement for obtaining the eye characteristics and the photographing for obtaining the image of the subject eye E. The objective examinations include the refractive power measurement (refraction measurement), the corneal shape measurement (keratometry measurement), the intraocular pressure measurement, the ocular fundus photographing, the tomogram imaging using optical coherence tomography (OCT) (OCT imaging), and the measurement using OCT. The subjective examination presents a target to an examinee and measures information (eye characteristics) on the subject eye E based on the responses from the examinee to the presented target. The subjective examinations include subjective refraction measurements such as the far-point examination, the intermediate-point examination, the near-point examination, the contrast examination, and the glare examination; and the visual field examination.

The entire configuration of the ophthalmologic apparatus 100 of the first embodiment will be described with reference to FIG. 1.

As shown in FIG. 1, the ophthalmologic apparatus 1 of the first embodiment includes a support base 110, a measurement unit 120, an examiner's control device 130, and a controller 140. In the following, as seen from the examinee, a left and right direction (horizontal direction) is defined as an X direction, an up and down direction (vertical direction) is defined as a Y direction, and a direction orthogonal to the X and Y direction (depth directions) is defined as a Z direction.

The support base 110 includes a column 111 rising from a floor and an optometric table 112 supported by the column 111. The optometric table 112 is a base for supporting a posture of the examinee. The optometric table 112 is also used to place a device and a tool for use in optometry, such as the examiner's control device 130, thereon. The optometric table 112 may be fixed at a position (height position) in the Y direction or may be supported on the column 111 to adjust the position (height position) of the optometric table 112 in the Y direction.

The measurement unit 120 includes an arm 121, a measurement head 122, and a forehead rest 123. One end of the arm 121 is supported by an end portion of the column 111, and the other end of the arm 121 extends toward a near side (the examinee's side) from the column 111 in the Z direction. The measurement head 122 is attached to an end of the arm 121. The measurement head 122 is thereby hung on the column 111 through the arm 121 above the optometric table 112. The arm 121 is configured to be move in the Y direction relative to the column 111. The arm 121 may be configured to be move in the X and Z directions relative to the column 111.

The measurement head 122 is configured to measure the eye characteristics of the subject eyes E. The measurement head 122 includes a driver 122a and a pair of a left measurement portion 122L and a right measurement portion 122R, which are provided under the driver 122a. The left and right measurement portions 122L, 122R are configured to measure the left and right eyes of the examinee, respectively.

The left measurement portion 122L includes a left measurement optical system 125L that presents a visual target to the left eye of the subject eye E of the examinee (hereinafter referred to as the "left subject eye") and measures the eye characteristics of the left subject eye under a condition adjusted at an any spherical power (corrected power). The right measurement portion 122R includes a right measurement optical system 125R that presents a visual target to the right eye of the subject eye E of the examinee (hereinafter referred to as the "right subject eye") and that measures eye characteristics of the right subject eye under a condition adjusted at an arbitrary spherical power (corrected power). The measurement results (objective measurement information) obtained by the left and right measurement optical systems 125L, 125R will be input to the controller 140. Detailed configurations of the left and right measurement optical systems 125L, 125R are described hereinafter.

The driver 122a allows each of the left and right measurement portions 122L, 122R to move horizontally (in the X direction) and vertically (in the Y direction) and to rotate in the X and Y directions.

The forehead rest 123 is provided in the measurement unit 120 and is disposed between the left and right measurement portions 122L, 122R. The forehead rest 123 supports the face of the examinee when the examinee places a part (forehead) of his or her face in contact with the forehead rest 123 during the measurement of the eye characteristics. In other words, the examinee facing towards the optometric table 112 can press his or her forehead against the forehead rest 123 so that he or she could stabilize the direction and the position of the face. The position of the forehead rest 123 can be adjusted by moving the arm 121 in the Y direction relative to the column 111.

The examiner's control device 130 is an information processing device that receives input operations by the examiner and outputs control signals to the controller 140. The examiner's control device 130 can be implemented with a portable device such as a tablet terminal or a smartphone, which can be separated from the measurement unit 120 so that the examiner can carry the examiner's control device 130. The examiner's control device 130 may be implemented with a personal computer such as a laptop personal computer or a desktop personal computer. The examiner's control device 130 may be implemented with a dedicated controller provided in the ophthalmologic apparatus 100. The examiner's control device 130 can exchange information with the controller 140 via wireless communication and/or network communication.

As shown in FIG. 1, the examiner's control device 130 includes an operation controller (not shown), a display 131, and input buttons 132 (see FIG. 3). The display 131 is a touch panel display provided on the examiner's control device 130. The display 131 may be configured to display the input buttons 132. The operation controller is a microcomputer built in the examiner's control device 130. The operation controller is configured to control images displayed on the display 131 based on the measurement results and/or the detection results sent from the controller 140. The operation controller outputs the control signals to the controller 140 according to the operation to the input buttons 132.

The detailed configurations of the left and right measurement optical systems 125L, 125R will be described with reference to FIG. 2. Since the left and right measurement optical systems 125L, 125R have the same configuration, only the left measurement optical system 125L will be described, and the description of the right measurement optical system 125R will be omitted in the present specification.

The left measurement optical system 125L includes a Z-alignment system 1, an XY-alignment system 2, a keratometory measurement system 3, a visual target projection system 4, an anterior ocular segment observation system 5, a refraction measurement projection system 6, and a refraction measurement light receiving system 7 so that the left measurement optical system 125L can work as an optical system for presenting a visual target to the subject's eye E (subject's left eye) and examining the subject's eye E. In the following, "ocular fundus conjugate position" means position optically and substantially conjugate with an ocular fundus Ef of the subject eye E, which can include an exact position optically conjugate with the ocular fundus Ef of the subject eye E and any proximal positions near the exact position, in an aligned state. "Pupil conjugate position" means position optically and substantially conjugate with a pupil of the subject eye E, which can include an exact position optically conjugate with the pupil of the subject eye E and any proximal positions near the exact position, in an aligned state.

The anterior ocular segment observation system 5 is configured to capture images of the anterior ocular segment of the subject eye E. The optical system that uses at least a part of the anterior ocular segment observation system 5 has an imaging face of an imaging element 59 at the pupil conjugate position Q. An anterior ocular segment illumination light source 51 is configured to illuminate light (e.g., infrared light) onto the anterior ocular segment of the subject eye E. The light reflected from the anterior ocular segment of the subject eye E passes through an objective lens 52, is transmitted through a dichroic mirror 53 and a half mirror 54, passes through a relay lens 55 and a relay lens 56, and is transmitted through a dichroic mirror 57. The light transmitted through the dichroic mirror 57 forms an image on the imaging surface of the imaging element 59 (area sensor) by using the imaging lens 58. The imaging element 59 captures images to output a signal at a predetermined rate. The output (image signal) of the imaging element 59 is input to the controller 140. The controller 140 displays the anterior ocular segment image, which is based on the image signal as an output of the imaging element 59, on the display 131 of the examiner's control device 130. The anterior ocular segment image is, for example, an infrared image or moving video.

The Z-alignment system 1 is configured to project light (infrared light) onto the subject eye E for the alignment purpose in the direction of the optical path (front or back direction, Z direction) of the anterior ocular segment observation system 5. The light from the Z-alignment light source 11 is turned into a parallel light flux by using the projecting lens 12 and then is projected onto the cornea of the subject eye E through an alignment hole formed through a keratometric plate 31. Based on a bright spot projected onto the cornea, the controller 140 or the examiner moves the left measurement optical system 125L in the Z direction by controlling the driver 122a such that the ratio of the distance between two spot images by the Z-alignment light source 11 on the imaging element 59 and the diameter of the keratometric ring image should fall in a predetermined range.

The XY-alignment system 2 is configured to illuminate light (infrared light) onto the subject eye E for the alignment purpose in the directions (left or right direction (X direction) and up or down direction (Y direction)) orthogonal to the optical axis of the anterior ocular segment observation system 5. The XY-alignment system 2 includes an XY-alignment light source 21 and a projecting lens 22. The XY-alignmentlight source 21 is provided in an optical path branched from the anterior ocular segment observation system 5 by using the half mirror 54. The light from the XY-alignmentlight source 21 is transmitted through the projecting lens 22, is reflected by the half mirror 54, and then is projected onto the subject eye E through the anterior ocular segment observation system 5. The reflection light by the cornea of the subject eye E is guided to the imaging element 59 through the anterior ocular segment observation system 5.

The alignment method in each of XYZ directions is not limited to the method using the Z-alignment system 1 and the XY-alignment system 2. The alignment methods can be ones capable of measuring the position of the subject eye E, for example, by using a stereo camera in the ophthalmologic apparatus 100.

The anterior ocular segment image includes the image (bright spot image) based on the reflection light from the cornea. The controller 140 displays the anterior ocular segment image including the bright spot image and the alignment mark on the display 131. With the manual XY alignment, the examiner's control device 130 controls the driver 122a to guide the bright spot image within the alignment mark and move the left measurement optical system 125L in the X and Y directions. With the automatic XY-alignment, the controller 140 controls the driver 122a to cancel the displacement of the bright point image relative to the alignment mark and move the left measurement optical system 125L in the X and Y directions.

The keratometry measurement system 3 is configured to project a ring-shaped light flux (infrared light) onto the cornea for measuring the shape of the cornea of the subject eye E. The keratometric plate 31 is disposed between the objective lens 52 and the subject eye E. A keratometric ring light source 32 is provided on the back side (side of the objective lens 52) of the keratometric plate 31. Illuminating the keratometric plate 31 with the light from the keratometric ring light source 32 projects the ring-shaped light flux on the cornea of the subject eye E. The imaging element 59 detects the reflection light (keratometric ring image) from the cornea of the subject eye E together with the anterior ocular segment image. The controller 140 calculates the cornea shape parameter representing the shape of the cornea with a known calculation based on the keratometric ring image.

The visual target projection system 4 presents various visual targets, such as fixation targets and visual targets for the subjective examination, to the subject eye E. The visual target projection system 4 includes a display 41, a half mirror 42, a relay lens 43, a reflective mirror 44, a focusing lens 45, a relay lens 46, a field lens 47, a variable cross-cylinder lens (VCC) 48, a reflective mirror 49, and a dichroic mirror 68. The visual target projection system 4 shares the dichroic mirror 53 and the objective lens 52 with the anterior ocular segment observation system 5. The visual target projection system 4 includes at least two glare light sources 41a, which illuminate the subject eye E with glare light, at a position around the optical axis and on an optical path different from the optical path to the display 41 for the subjective examination.

The light output from the display 41 is reflected by the half mirror 42, is transmitted through the relay lens 43, is reflected by the reflective mirror 44, and is transmitted through the focusing lens 45. The light transmitted through the focusing lens 45 is transmitted through the relay lens 46, and the travel direction of the light is aligned by the field lens 47. Then, the light is transmitted through the VCC 48, is reflected by the reflective mirror 49, is transmitted through the dichroic mirror 68, and then is reflected by the dichroic mirror 53. The light reflected by the dichroic mirror 53 passes through the objective lens 52 and is irradiated onto the ocular fundus Ef.

The display 41 displays the fixation target or the point-like visual target as the visual target for fixing a line of sight during the objective examination or fogging to the subject eye E. The display 41 also displays the subjective examination visual target for subjectively examining eye characteristics (e.g., visual acuity value, far-point power, and near-point power) of the subject eye E. The display 41 can be implemented with an organic electroluminescence (EL) display or a liquid crystal display (LCD). The display 41 displays images under the control of the controller 140. The display 41 is provided at a position P conjugate with the fundus (also referred to as fundus conjugate position P hereinafter) in the optical path of the visual target projection system 4.

The controller 140 controls a drive motor (not shown) to drive the focusing lens 45 to move forward or backward in the optical axis direction. The controller 140 moves the focusing lens 45 toward the subject eye E to displace the spherical power of the subject eye E to the negative diopter side. Also, the controller 140 moves the focusing lens 45 in a direction away from the subject eye E to displace the spherical power of the subject eye E to the positive diopter side. Furthermore, the forward or backward movement of the focusing lens 45 changes the position of presenting the visual target displayed on the display 41, so that the controller 140 changes the examination distance from the presentation position of the visual target to the subject eye E.

For the subjective examination, the controller 140 controls the focusing lens 45 to move in the optical axis direction based on the result of the objective examination, thereby controlling the examination distance or the spherical power of the subject eye E. The controller 140 displays, on the display 41, a predetermined visual target, which has been selected by the examiner or the controller 140, to present the visual target to the examinee at a predetermined examination distance relative to the subject eye E adjusted to having a predetermined spherical power. The controller 140 may further control or calculate a subjective examination value by receiving the input of the response of the examinee to the visual target. For example, in the visual acuity measurement, the controller 140 selects and displays the next visual target based on the response to the visual target such as the Landolt ring and determines the visual acuity value by repeating this process.

The refraction measurement projection system 6 and the refraction measurement light receiving system 7 constitute an objective measurement optical system for use in objective refraction measurements (refraction measurements) that objectively measure refraction values as eye characteristics of the left and right subject eyes, respectively. The refraction measurement projection system 6 projects a ring-shaped light flux (infrared light) for objective measurement onto the ocular fundus Ef. The refraction measurement light receiving system 7 receives the return light from the subject eye E of the ring-shaped light flux.

A refraction measurement light source 61 may be implemented with a super luminescent diode (SLD) light source, which is a high-intensity light source with an emission diameter less than a predetermined size. The refraction measurement light source 61 is configured to move in the optical axis direction and is disposed at the ocular fundus conjugate position P. A ring diaphragm 65 (specifically, which has a transmissive portion) is disposed at the pupil conjugate position Q. A focusing lens 74 is configured to move in the optical axis direction. The focusing lens 74 may be a known variable focusing lens, which can change its focal position under the control of the controller 140. The optical system via the refraction measurement light receiving system 7 disposes the imaging surface of the imaging element 59 at the ocular fundus conjugate position P. The refraction measurement light source 61 and the focusing lens 74 are configured to move in conjunction in their optical axis direction. Furthermore, in the first embodiment, the refraction measurement light source 61, the focusing lens 74, and the focusing lens 45 of the visual target projection system 4 are configured to move in their optical axes in conjunction with each other.

The light from the refraction measurement light source 61 passes through a relay lens 62 and enters onto a cone surface of a cone prism 63. The light incident on the cone surface is deflected and emitted from the bottom surface of the cone prism 63. The light emitted from the bottom surface of the cone prism 63 passes through a field lens 64 and through the transmissive portion formed in a ring shape on the ring diaphragm 65. The light (the ring-shape light flux) that has passed through the transmissive portion of the ring diaphragm 65 is reflected by a reflection surface of a holed prism 66, passes through a rotary prism 67, and is reflected by the dichroic mirror 68. The light reflected by the dichroic mirror 68 is reflected by the dichroic mirror 53, passes through the objective lens 52, and is projected onto the subject eye E. The rotary prism 67 averages and/or equalizes the light volume distribution of the ring light flux over disease sites and/or blood vessels in the ocular fundus Ef to reduce the speckle noise caused by the light source.

It is preferable to dispose the cone prism 63 as close as possible to the pupil conjugate position Q.

The field lens 64 may include the ring diaphragm 65 affixed to the lens surface on the side of the subject eye E, for example. In this case, a light shielding film may be deposited on the lens surface of the field lens 64 so that a ring-shaped transmissive portion is formed. The refraction measurement projection system 6 may have a configuration in which the field lens 64 is omitted.

The cone prism 63 may include a ring diaphragm 65 affixed to the bottom surface that faces the field lens 64, for example. In this case, a light shielding film may be deposited on the bottom surface of the cone prism 63 so that a ring-shaped transmissive portion is formed. The ring diaphragm 65 may be disposed on the side of the cone surface of the cone prism 63.

The ring diaphragm 65 may consist of a diaphragm with a transmissive portion having a shape corresponding to a predetermined measurement pattern. This diaphragm may include a transmissive portion formed at a position eccentric to the optical axis of the refraction measurement projection system 6. The diaphragm may include two or more transmissive portions.

The return light of the ring-shaped light flux projected onto the ocular fundus Ef passes through the objective lens 52 and is reflected by the dichroic mirror 53 and the dichroic mirror 68. The return light reflected by the dichroic mirror 68 passes through the rotary prism 67, passes through the hole in the holed prism 66, passes through the relay lens 71, is reflected by a reflective mirror 72, and passes through a relay lens 73 and the focusing lens 74. The return light that has passed through the focusing lens 74 is reflected by the reflective mirror 75, reflected by the dichroic mirror 57, and forms an image on the imaging surface of the imaging element 59 by the imaging lens 58. The controller 140 calculates the parameter of the ocular refractive power by using any known calculation, based on the output from the imaging element 59. The parameter of the ocular refractive power includes refractive value (refractive power), spherical power, astigmatism power, and astigmatic axis angle of the subject eye E.

The controller 140 is implemented by an information processing device, which is provided under the optometric table 112. Based on the control signals from the examiner's control device 130, the controller 140 comprehensively controls the left and right measurement optical systems 125L, 125R (each of which includes the refraction measurement projection system 6, the refraction measurement light receiving system 7, the visual target projection system 4), and the measurement unit 120 (which includes the driver 122a). The controller 140 sends the measurement results of the eye characteristics of the subject eye E measured by the measurement head 122 to the examiner's control device 130.

The controller 140 includes one or more computer processors, which may be implemented by using circuitry such as a Central Processing Unit (CPU), Graphics Processing Unit (GPU), Application Specific Integrated Circuit (ASIC), or a programmable logic device (e.g., Simple Programmable Logic Device (SPLD), Complex Programmable Logic Device (CPLD) or Field Programmable Gate Array (FPGA)). For example, the controller 140 performs desired functions by reading out and executing the program stored in a storage circuit or storage device.

As shown in FIG. 3, the controller 140 includes a main controller 141, a storage 142, and a calculation processor 143.

The main controller 141 controls the Z-alignment light source 11 of the Z-alignment system 1, the XY-alignment light source 21 of the XY-alignment system 2, and the keratometric ring light source 32 of the keratometry measurement system 3, thereby changing the amount of the light output from the Z-alignment light source 11, the XY-alignment light source 21, and/or the keratometric ring light source 32 and switching them on or off.

The main controller 141 controls the display 41 of the visual target projection system 4, thereby switching on or off the display of the visual target and fixation target shown on the display 41 and switching the visual target and fixation target alternately.

The main controller 141 controls the imaging element 59 and the anterior ocular segment illumination light source 51 of the anterior ocular segment observation system 5, thereby changing the amount of the light output from the anterior ocular segment illumination light source 51 and switching it on or off. The main controller 141 changes the detection sensitivity by changing the exposure time of the imaging element 59 and by controlling the gain of the amplifier in the imaging element 59. The signal acquired by the imaging element 59 is taken into the controller 140, thereby the calculation processor 143 forms images, etc.

The main controller 141 controls the refraction measurement light source 61 and the rotary prism 67 of the refraction measurement projection system 6 and the focusing lens 74 of the refraction measurement light receiving system 7, thereby changing the amount of the light output from the refraction measurement light source 61 and switching it on or off. The main controller 141 changes the rotation speed of the rotary prism 67 and switches the rotation operation on or off. Also, the main controller 141 changes the position of the focusing lens 74 in the optical axis direction. The main controller 141 controls a movement mechanism (not shown) to move the refraction measurement light source 61 and the focusing lens 74 in the optical axis direction. The main controller 141 controls the movement mechanism to move the refraction measurement light source 61 and the focusing lens 74 in the optical axis direction, to put the ocular fundus Ef, the refraction measurement light source 61, and the imaging surface of the imaging element 59 optically conjugated, based on the calculated refraction power value. When the focusing lens 74 consists of a variable focus lens, the main controller 141 changes the focus position of the focusing lens 74 by controlling the focusing lens 74.

In the ophthalmologic apparatus 100 of the first embodiment, the main controller 141 controls a movement mechanism (not shown) to move the focusing lens 45 of the visual target projection system 4. The movement mechanism includes an actuator that produces a driving force for the movement and a transmission mechanism for transmitting the driving force. The actuator includes, for example, a pulse motor. The transmission mechanism includes elements such as a combination of gears or a rack and pinion. The main controller 141 controls the movement mechanism to move the focusing lens 45 in the optical axis direction by controlling such a movement mechanism. The ophthalmologic apparatus 100 changes the examination distance and the spherical power (corrected power) of each of the left and right subject eyes E by moving the focusing lens 45 in the optical axis direction.

The main controller 141 writes in data in the storage 142 and reads out the data from the storage 142.

Furthermore, the main controller 141 of the first embodiment is configured to execute an examination for the subject eyes E. During the examination, the fixation targets are respectively presented to the left and right subject eyes E under a condition that the left and right subject eyes E are in anisometropia, then the refraction values of the left and right subject eyes E are measured while the examination distance from the fixation targets to the left and right subject eyes E is changed within a predetermined distance range, and then based on the measured refraction values, the fixation eye is determined. Detailed procedures of the method of this examination will be described hereinafter.

The storage 142 stores various data. The data stored in the storage 142 include information and data such as the measurement information acquired by the keratometry measurement system 3, the measurement information acquired by the refraction measurement projection system 6 and the refraction measurement light receiving system 7, the image data of the images acquired by the imaging element 59, and the subject eye information. The subject eye information includes information regarding an examinee, such as an ID and name, and information regarding the subject eyes such as identification information of left and right eyes. The storage 142 stores the measurement information acquired by the keratometry measurement system 3 when the ophthalmologic apparatus 100 performs the keratometry measurement of the subject eye E. The storage 142 stores the measurement information acquired by the refraction measurement projection system 6 and the refraction measurement light receiving system 7 when the ophthalmologic apparatus 100 performs the refraction measurement of the subject eye E. The storage 142 may be used as a working memory of the calculation process of the corneal shape parameter and the calculation process of the refraction value of the subject eye E. The storage 142 stores various programs and data for operating the ophthalmologic apparatus 100.

The calculation processor 143 executes various calculations for determining parameters showing eye characteristics of the subject eye E, such as the corneal shape parameter and the refractive power parameter. The calculation processor 143 of the first embodiment includes an analyzer 144 and a fixation eye determiner 145.

The analyzer 144 analyzes the ring image (pattern image) obtained by the imaging element 59 receiving the return light of the ring light flux (ring measurement pattern) projected onto the ocular fundus Ef from the refraction measurement projection system 6. For example, the analyzer 144 calculates the geometric center of the ring image based on the brightness distribution of the image the ring image is obtained, analyses the brightness distribution along a plurality of scanning directions radially extending from the geometric center, and specifies the ring image based on the brightness distribution. The analyzer 144 determines whether or not the brightness of the specified ring image (ring brightness) is within a predetermined brightness range. The analyzer 144 may determine whether or not an S/N ratio of the specified ring image is within a predetermined numerical value range.

The analyzer 144 determines the spherical power, the astigmatism power, and the astigmatism axis angle by determining an approximate ellipse of the specified ring image and substituting the long diameter and the short diameter of the approximate ellipse into a known formula. Furthermore, the analyzer 144 determines the refractive value, the spherical power, the astigmatism power, and the astigmatism axis angle of the subject eye E, based on the specified ring image (approximate ellipse) and the control content (e.g., movement amount) to the focusing lens 74. Alternatively, the analyzer 144 can determine the parameter of the eye refractive power such as the refractive value of the subject eye E based on the deformation and displacement of the ring image relative to the reference or standard pattern.

The analyzer 144 calculates the corneal shape parameters such as the corneal refractive power, the corneal astigmatism power, and the corneal astigmatism angle based on the keratometric ring image acquired by the anterior ocular segment observation system 5. For example, the analyzer 144 calculates the corneal curvature radii of the steeper meridian and flatter meridian of the corneal front face by analyzing the keratometric ring image and calculates the corneal shape parameters based on the corneal curvature radii.

The fixation eye determiner 145 determines the fixation eye based on the refractive values of the subject eyes E, which is measured with the objective measurement. Here, the term "fixation eye" refers to one of the subject person's eyes, either the left or right, that the subject person uses primarily to see (or fixates) an object mainly and to focus the object substantially at the fovea centralis. Generally, when both left and right subject eyes E are maintained in a fully corrected condition, both left and right subject eyes E establish visual fixation, thereby causing both eyes to become fixation eyes. Or, when the left and right subject eyes E are under an anisometropia condition, one of the left and right subject eyes E becomes the fixation eye.

Also, the term "fully corrected condition" refers to a state of the eye where the eye subjectively experiences no refraction abnormality, which is achieved with a prescription of the fully corrected power with the minimum load power to attain the best visual acuity. The term "anisometropia condition" refers to a state of the eye where the left and right subject eyes E exhibit displacement of their refractive condition from the visual target position. In the first embodiment, the "anisometropia condition" means a condition where one of the left and right subject eyes E is maintained in the fully corrected condition and the other subject eye E is different in spherical power by a predetermined power (e.g., +2 diopters, also referred to as +2D hereinafter) from the fully corrected condition, thereby causing the difference of the predetermined power (e.g., 2D) or more between the refractive values (spherical powers) of the left and right subject eyes E.

Specifically, the fixation eye determiner 145 determines whether the subject eye E can be the fixation eye by using, for example, the following fixation eye conditions. (1) The subject eye with a difference in refractive value that is equal to or lower than a predetermined threshold, in which the difference is measured between a refractive value of the subject eye itself and a refractive value at the presentation position of the fixation target when the fixation target is presented. (2) The subject eye E, among the left and right subject eyes E, whose refractive value more closely matches a refractive value at the presentation position of the fixation target when the examination distance from the fixation target to the subject eye E has changed from the far-point vision to the near-point vision (the subject eye E, among the left and right subject eyes E, that causes a larger decrease in the difference between the refractive value of the subject eye itself and the refractive value at the presentation position of the fixation target when the examination distance has changed from the far-point vision to the near-point vision).

The procedure of the examination method of the subject eyes E using the ophthalmologic apparatus 100 of the first embodiment will be explained as follows with reference to the flowchart shown in FIG. 4.

In Step S1, the main controller 141 (controller 140) controls the visual target projection system 4, the anterior ocular segment observation system 5, the refraction measurement projection system 6, and the refraction measurement light receiving system 7 to capture the ring image and, based on the captured ring image, executes "preliminary measurement", which measures the eye characteristics including the refractive values of the left and right subject eyes E. Then, the process proceeds to Step S2. The main controller 141 may execute the preliminary measurement of Step S1 two or more times to determine the measurement condition of the same type or those of different types by executing each of the preliminary measurement so that the measurement condition should be converged each time while the preliminary measurements are repeated. For example, each of the preliminary measurement may determine at least one of the control details for the light intensity of the light output from the refraction measurement light source 61, the exposure time of the imaging element 59, the detection sensitivity of the imaging element 59, and the focusing lens 74 based on the obtained ring image.

In Step S2, following the preliminary measurement in Step S1, the main controller 141 executes the "main measurement", which measures the predetermined target eye characteristic (the refractive values of the subject eyes E in this embodiment) under the measurement condition determined by the last one of the preliminary measurements. Then, the process proceeds to Step S3. The following procedure determines the refractive value of the subject eye E. The main controller 141 controls the refraction measurement projection system 6 to project the ring-shaped light flux (infrared) for the objective measurement onto the ocular fundus Ef of the subject eye E. Then, the main controller 141 controls the refraction measurement light receiving system 7 to detect (receive) the return light of the ring-shape light flux from the ocular fundus Ef and acquires the refractive value of the subject eye E by a known method based on the image signals detected with the imaging element 59. Step S1 and Step S2 each is a process of the objective examination.

In Step S3, following the main measurement in Step S2, the main controller 141 sets the start of the subjective examination (also referred to as subjective examination start setting) for each of the left and right subject eyes E. Then, the process proceeds to Step S4. Here, the "subjective examination start setting" involves adjusting the positions of the focusing lenses 45, 45 respectively in the visual target projection systems 4, 4, each of which is of the left and right measurement optical systems 125L, 125R, to make the spherical powers of the left and right subject eyes E into fully corrected conditions at the predetermined positions for the far-point vision (e.g., examination distance = zero diopters (0D)) by using the calculation based on the refractive values of the subject eyes E obtained in the main measurement. The "diopter (D)" is defined as the reciprocal number of the focal length represented in meters, and accordingly, "examination distance = 0D" means that the examination distance is infinite.

In Step S4, following the subjective examination start setting in Step S3, the main controller 141 executes an RG examination for one eye at a time using an RG chart. The "RG chart" consists of several visual markers in different colors, such as red icons and green icons, which are arranged on the left and right respectively. For example, the visual markers may include numerical characters in different sizes; and/or a double-circled mark and/ a single-circled mark in different sizes. The "RG examination" refers to an examination that aims to subjectively check if the corrected condition of the subject eye E is the fully corrected condition (proper condition) (if it is over-corrected or under-corrected), using the RG chart, based on an optical characteristic known as chromatic aberration. Due to the characteristics of the ocular media of the eye, in which a shorter-wavelength light has more power at the refractive surface, a green-wavelength light forms an image at a position that is rather shifted toward the incident side than a red-wavelength light. Thus, the under-corrected condition in the subject eye E, which causes the eye to shift a focus point of the visual targets forward in front of the retina, and using a red-wavelength light, which causes a backward shift at the same time, allow the eye to see the red visual target more clearly. The full-corrected condition of the subject eye E allows the eye to see the green and red visual targets almost equally clearly. The over-corrected condition of the subject eye E causes the eye to see the green visual target more clearly. These mechanisms are used in the first embodiment, in which the spherical power (corrected power) of the subject eye E is adjusted by changing the position of the focusing lens 45 until the examinee equally sees the green and red visual targets of the RG chart, that is, until confirming the fully corrected conditions of the left and right subject eyes E. As a result, the left and right subject eyes E can achieve the fully corrected values.

In Step S5, following the RG examination in Step S4, or determining that the examination distance is not equal to the predetermined distance in Step S6, the main controller 141 adjusts the positions of the focusing lenses 45 and presents the fixation targets (left fixation target 500L and right fixation target 500R, which will be described later) at the predetermined positions for the far-point vision (e.g., examination distance = 0D) to the left and right subject eyes E, respectively. Furthermore, while presenting the fixation targets (left and right fixation targets 500L, 500R), the main controller 141 controls the moving mechanism to move the focusing lenses 45 in the optical axis directions, to change the presentation positions of the fixation targets (left and right fixation targets 500L, 500R) to the near-point vision by the same distance for both the left and right targets. Then, the process proceeds to Step S6. Thereby, this can shorten the examination distance under a condition that is achieved with the fully corrected values in the far-point vision. In the first embodiment, the main controller 141 continuously changes the presentation positions while presenting the fixation targets.

The visual target projection system 4 presents the different fixation targets to the left and right subject eyes E, respectively. In other words, the visual target projection system 4 of the left measurement optical system 125L (left-eye projection optical system) presents the left fixation target 500L shown in FIG. 5A to the left subject eye, while the visual target projection system 4 of the right measurement optical system 125R (right-eye projection optical system) presents the right fixation target 500R shown in FIG. 5B to the right subject eye.

The left fixation target 500L includes a background target 501L, a fusion target 502L, and an examination target 503L. The background target 501L is a visual target depicted, for example, in the entirety of a visual target presentation region, on which the fusion target 502L and the examination target 503R are superimposed. The background target 501L is depicted as a white rectangular shape. The fusion target 502L is a visual target that can be depicted in a manner that allows binocular fusion while the left subject eye views the left fixation target 500L and the right subject eye views the right fixation target 500R. The fusion target 502L is depicted in black at the center of the background target 501L. The examination target 503L is a visual target depicted not in a manner that allows binocular fusion while when the left subject eye views the left fixation target 500L and the right subject eye views the right fixation target 500R. The examination target 503L is depicted in black below the fusion target 502L.

The right fixation target 500R includes a background target 501R, a fusion target 502R, and an examination target 503R. The background target 501R is a visual target depicted, for example, in the entirety of a visual target presentation region, on which the fusion target 502R and the examination target 503R are superimposed. The background target 501R is depicted as a white rectangular shape. The fusion target 502R is a visual target that can be depicted in a manner that allows binocular fusion while the left and right subject eyes respectively view the left and right fixation targets 500L, 500R, and is depicted in black at the center of the background target 501R. The examination target 503R is a visual target depicted not in a manner that allows binocular fusion while the left and right subject eyes respectively view the left and right fixation targets 500L, 500R, and is depicted in black above the fusion target 502R.

If the examinee sees the left and right fixation targets 500L, 500R in the manner of the binocular fusion while the left subject eye views the left fixation target 500L and the right subject eyes view the right fixation target 500R, the examinee sees the background targets 501L, 501R as one target and the fusion targets 502L, 502R as one target and also sees the examination targets 503R, 503L above and below in the middle of the fusion targets 502L, 502R therebetween as shown in FIG. 5C. If the examinee sees the left and right fixation targets 500L, 500R in the manner of the binocular fusion, the main controller 141 may stop the examination for the subject eyes E shown in FIG. 4 or may issue an alert prompting the examiner to decide whether to continue the examination based on results input by the examinee.

In Step S6, after presenting the fixation targets and changing the target positions in Step 5, the main controller 141 determines whether or not the examination distance, which is the distance from the subject eye E to the fixation target (left fixation target 500L, right fixation target 500R), has reached a predetermined distance. If the main controller 141 determines YES (i.e., the examination distance has reached the predetermined distance), the process will proceed to Step S7. If the main controller 141 determines NO (i.e., the examination distance has not reached the predetermined distance), the process will return to Step S5. The predetermined distance is set at 4D in the first embodiment but may be set as desired.

As a result, the ophthalmologic apparatus 100 of the first embodiment can present the fixation targets (left and right fixation targets 500L, 500R) to the left and right subject eyes E under the fully corrected condition and can change the examination distances (presentation positions of the fixation targets (left and right fixation targets 500L, 500R)) in a predetermined distance range (e.g., 0D to 4D).

In Step S7, after determining that the examination distance has reached the predetermined distance in Step S6 or determining that the binocular examination has been stopped and returned in Step S15, the main controller 141 controls the movement mechanism to move the focusing lenses 45 in the optical axis direction and adjusts again the spherical powers of the left and right subject eyes E, respectively, into the fully corrected condition. Then, the process proceeds to Step S8.

In Step S8, following the adjustment of the spherical powers in Step 7, the fixation targets (the after-mentioned left and right fixation targets 500L, 500R) are respectively presented to the left and right subject eyes E at a predetermined far-point vision position (e.g., examination distance = 0D) by adjusting the positions of the focusing lenses 45. Furthermore, the main controller 141 controls the movement mechanism to move the focusing lens 45 facing at one of the subject eyes E in the optical axis direction to change the spherical power of the one subject eye E by a predetermined power (e.g., addition of +2D) relative to the power in the fully corrected condition in the far-point vision. Then, the process proceeds to Step S9. At this time, the focusing lens 45 facing at the other subject eye E should not have been moved, thereby maintaining the spherical power of the other subject eye E in the fully corrected condition set in Step S7. As a result, since this allows the one subject eye E to become the corrected condition by a predetermined power (e.g., 2D) and the other subject eye E to become the fully corrected condition, this induces an anisometropia in the subject eyes E. The subject eye E of which spherical power is changed in Step S8 is the subject eye of which spherical power has not been changed during the test shown in FIG. 4.

In Step S9, after changing the spherical power of the one eye in Step S8 or determining that the examination distance has been changed by the distance of the subjective examination in Step S11, the examiner asks the examinee one or more questions about how the examination targets 503L, 503R are seen (which of the examination targets 503L, 503R is seen more clearly). Then, the process proceeds to Step S10. The examiner determines which one the fixation eye is, based on the examinee's answers. The examiner may input the examinee's answers into the fixation eye determiner 145 of the controller 140. When the main controller 141 determines that the examination distance has changed by the distance of the subjective examination, the main controller 141 temporarily suspends the change of the visual target position. The main controller 141 controls the movement mechanism to lock the presentation position of the fixation target during the time taken from the examiner's questions to the examinee's answers. After obtaining the examinee's answers, the main controller 141 resumes and controls the movement mechanism to changes the visual target position to the near-point vision, based on the input of the information such as the examinee's answers and the examiner's instructions.

In Step S10, after the inquiries to the examinee in Step S9 and determining that the examination distance has not reached the predetermined distance in Step S12, the main controller 141 the movement mechanism to move the focusing lenses 45 in the optical axis directions to change the presentation positions of the fixation targets (left and right fixation targets 500L, 500R), which are presented to the left and right subject eyes E, to the direction of the near-point vision by the same distance. Then, the process proceeds to Step S11. Thereby, this can shorten the examination distance under the condition of the anisometropia. In the first embodiment, the main controller 141 continuously changes the presentation positions while presenting the fixation targets.

In Step S11, after changing the visual target position in Step S10, the main controller 141 determines whether or not the examination distance has changed by a predetermined distance for the subjective examination. When the main controller 141 determines YES (i.e., the examination distance has changed by the predetermined distance for the subjective examination), the process returns to Step S9. When the main controller 141 determines NO (i.e., the examination distance has not changed by the predetermined distance), the process proceeds to Step S12. The predetermined distance for the subjective examination is set at +1D in the first embodiment. However, the subjective examination distance may be set as desired.

In Step 12, after determining that the examination distance has not changed by the subjective examination distance in Step S11, the main controller 141 determines whether or not the examination distance has reached a predetermined distance. When the main controller 141 determines YES (i.e., the examination distance has reached the predetermined distance), the process proceeds to Step S13. When it determines NO (i.e., the examination distance has not reached the predetermined distance), the process returns to Step S10. The predetermined distance is set at 4D in the first embodiment. However, the predetermined distance may be set as desired.

As a result, the ophthalmologic apparatus 100 of the first embodiment respectively presents the fixation targets (left and right fixation targets 500L, 500R) to the left and right subject eyes E under the anisometropia condition, and changes the examination distance in a predetermined range (0D to 4D) while the examiner asks the examinee one or more questions as to how the examination targets 503L, 503R are seen each time the examination distance changes by +1D.

In Step S13, after the determination that the examination distance has reached the predetermined distance in Step S12, the fixation eye determiner 145 determines the fixation eye based on the refractive values of the subject eyes E. Then, the process proceeds to Step S14. In other words, the fixation eye determiner 145 monitors the refractive values of the left and right subject eyes E during the time taken from presenting the fixation targets under the fully corrected condition and changing the visual target position in Step S5 to the determination that the examination distance has reached the predetermined distance in Step S12. Then, the fixation eye determiner 145 compares the refractive value according to the presentation position of the fixation target with each of the refractive values of the left and right subject eyes E. The fixation eye determiner 145 determines the fixation eye based on whether or not the subject eye E satisfies fixation eye conditions that set in advance; and the non-fixation eye based on whether or not the subject eye E does not satisfy the fixation eye conditions. The fixation eye determiner 145 may determine the fixation eye at the timing when the examination distance has reached a predetermined subjective examination distance or may continuously determine the fixation eye while the examination distance is changed. When determining the fixation eye, the fixation eye determiner 145 may consider the answer (result of the subjective examination) to the question to the examinee in Step S9.

In Step S14, after determining the fixation eye in Step S13, the main controller 141 determines whether or not the fixation switching has occurred while the examination distance changes in a predetermined distance range (0D to 4D) based on the determination result of the fixation eye in Step S13. Then, the process proceeds to Step S15. The "fixation switching" refers to the switching of the fixation eye as the examination distance changes.

In Step S15, after determining the fixation switching in Step S14, the main controller 141 determines whether or not the examination of the left and right subject eyes E has been completed. When the main controller 141 determines YES (i.e., the examination of both eyes has been completed), the process proceeds to END. When the main controller 141 determines NO (i.e., the examination of both eyes has not been completed), the process proceeds to Step S7. In Step S15, when the spherical power in Step S8 has been changed for both the left and right subject eyes E, it is determined that the examination for the left and right subject eyes E has been completed. In Step S15, when the spherical power in Step S8 has been changed only for either one of the left and right subject eyes E, it is determined that the examination for the left and right subject eyes E has not been completed (i.e., the examination of both eyes has not been completed).

In Step S16, the main controller 141 controls the refraction measurement projection system 6 and the refraction measurement light receiving system 7 to conduct the refraction measurement (measurement of refractive values) of the left and right subject eyes E. Then, the process proceeds to Step S17. The refraction measurement is conducted in real time in sequential order with the change of the examination distance during the time taken from changing the visual target position after the RG examination (Step S5) to determining that the examination distance has reached the predetermined distance (Step S12). The refraction measurement may be conducted continuously or intermittently at any timing during the time taken from changing the visual target position after the RG examination (Step S5) to determining that the examination distance has reached the predetermined distance (Step S12).

As a result, the main controller 141 acquires the refractive values of the left and right subject eyes E as the objective measurement information. The main controller 141 may intermittently acquire the objective measurement information (refractive values) at the timing when the examination distance has reached a predetermined distance (e.g., timing when the examination distance has changed by the subjective examination distance (e.g., +1D)). In this case, the main controller 141 may temporarily suspend the change of the examination distance during the period to acquire the objective measurement information, that is, to measure the refractive values. Furthermore, the main controller 141 may continuously acquire the objective measurement information (refractive values) during the period while the examination distance is being changed in a predetermined distance range (0D to 4D).

In Step S17, following the refraction measurement in Step S16, the main controller 141 outputs and issues a command to the examiner's control device 130 to display the objective measurement information (refractive values) on the display 131 at a predetermined timing. As a result, the objective measurement information (refractive values) is displayed on the display 131 at a predetermined timing. The objective measurement information displayed on the display 131includes information that represents the relationship of the refractive values of the left and right subject eyes E to the examination distance in a two-dimensional coordinate graph (see FIGS. 6A to 8C). The "predetermined timing" may be, for example, a timing when the examination distance has changed by the subjective examination distance (e.g., +1D). The refractive value may be displayed in real-time at each measurement. The refractive value to be displayed may be acquired at the timing of display or may be an average value among values acquired during a certain period of time.

The operation of the ophthalmologic apparatus 100 of the first embodiment is described below.

The ophthalmologic apparatus 100 of the first embodiment conducts the preliminary measurement (Step S1) and the main measurement (Step S2). Subsequently, the ophthalmologic apparatus 100 adjusts the spherical power until each of the left and right subject eyes E is in a fully corrected condition (Steps S3 and S4). Next, the ophthalmologic apparatus 100 respectively presents the left and right fixation targets 500L, 500R to the left and right subject eyes with the left and right subject eyes E being maintained in the fully corrected condition while changing the examination distance in a predetermined distance range (0D to 4D). Then, the ophthalmologic apparatus 100 measures the refractive values of the left and right subject eyes E that binocularly view the fixation targets (left and right fixation targets 500L, 500R) to acquire the objective measurement information (Step S5, Step S6, Step S16, and Step S17: the after-mentioned third step).

In other words, the ophthalmologic apparatus 100 of the first embodiment respectively presents the fixation targets (left and right fixation targets 500L, 500R) to the left and right subject eyes E in the fully corrected condition at a predetermined far-point vision position (examination distance = 0D) and changes the presentation position of the fixation targets (left and right fixation targets 500L, 500R) to a near-point vision direction while presenting the fixation targets (left and right fixation targets). Then, the ophthalmologic apparatus 100 measures the refractive values of the subject eyes E that view the fixation targets (left and right fixation targets 500L, 500R). In the first embodiment, the ophthalmologic apparatus 100 measures the refractive values of the left and right subject eyes E at each 1D increment of the examination distance within the range of 0 to 4D.

It is possible to calculate the amount of an adjustment reaction of each subject eye E by the amount of change of the refractive value with the change of the examination distance. Accordingly, the ophthalmologic apparatus 100 of the first embodiment objectively acquires the refractive values at examination distances of 0D, 1D, 2D, 3D, and 4D when both eyes are in the fully corrected condition, thereby measuring the adjustment reaction amounts of the left and right subject eyes E under their fully corrected condition. In other words, the measurement of the refractive values at different examination distances in the first embodiment is substantially equivalent to the measurement of the amount of the adjustment reaction.

Next, the ophthalmologic apparatus 100 of the first embodiment controls the movement mechanism to move the focusing lenses 45 in the optical axis direction to bring the spherical powers of the left and right subject eyes E to the fully corrected condition (Step S7). Subsequently, the ophthalmologic apparatus 100 maintains one of the subject eyes E (the left subject eye herein) in the fully corrected condition and changes the spherical power of the other subject eye E (the right side herein) from the power in the fully corrected condition by a predetermined power (addition of +2D) to induce an anisometropia with the addition of +2D only to the right subject eye E (Step S8). Thereby, the ophthalmologic apparatus 100 produces a monovision condition in which the left subject eye has been corrected for the far-point vision and the right subject eye has been corrected for the near-point vision.

In the anisometropia condition with the addition of +2D to the right subject eye, the ophthalmologic apparatus 100 of the first embodiment respectively presents the left fixation target 500L to the left subject eye E and the right fixation target 500R to the right subject eye E while changing the examination distance in a predetermined distance range (0D to 4D). Then, the ophthalmologic apparatus 100 measures the refractive values of the left and right subject eyes E, which binocularly view the fixation targets (the left and right fixation targets 500L, 500R) to acquire the objective measurement information (Step S10, Step S11, Step S12, Step S16, and Step S17: the after-mentioned first step).

In other words, the ophthalmologic apparatus 100 of the first embodiment respectively presents the fixation targets (left and right fixation targets 500L, 500R) to the left and right subject eyes E in the anisometropia condition with the addition of +2D to the right subject eye, at a predetermined far-point vision position (examination distance = 0D), and changes the presentation positions of the fixation targets (left and right fixation targets 500L, 500R) in the near-point vision direction while presenting the fixation targets (left and right fixation targets 500L, 500R). Then, the ophthalmologic apparatus 100 measures the refractive values of the subject eyes E viewing the fixation targets (left and right fixation targets 500L, 500R). In the first embodiment, the ophthalmologic apparatus 100 measures the refractive values of the left and right subject eyes E at each 1D increment of the examination distance within the range of 0 to 4D. Further, in the first embodiment, the examiner checks how the examinee views the left and right fixation targets 500L, 500R (subjective test) every time when the examination distance increases by a predetermined subjective examination distance (+1D) (every time when the examination distance reaches 0D, 1D, 2D, 3D and 4D) until the examination distance changes from 0D to 4D (Step S9).

Thus, the ophthalmologic apparatus 100 of the first embodiment measures the amount of the adjustment reaction of the left and right subject eyes E in the anisometropia condition with the addition of +2D only to the right subject eye from the fully corrected condition of both eyes and checks how the fixation targets are viewed at predetermined examination distances (0D, 1D, 2D, 3D and 4D).

Furthermore, after measuring the refractive values, the ophthalmologic apparatus 100 of the first embodiment determines the fixation eye (Step S13), based on the measured refractive values, each time when the examination distance reaches the predetermined subjective examination distance (each of 0D, 1D, 2D, 3D and 4D), in the anisometropia condition with the addition of +2D to the right subject eye. Based on the fixation eye determination result, the ophthalmologic apparatus 100 determines whether or not the fixation eye has switched (Step S14) while the examination distance changes in the anisometropia condition with the addition of +2D to the right subject eye. The display 131 suitably displays the fixation eye determination result and the result of determining whether or not the fixation eye has switched (fixation switching determination).

Further, after the fixation switching determination in the anisometropia condition with the addition of +2D to the right subject eye, the ophthalmologic apparatus 100 returns the spherical power of each of the left and right subject eyes E to the fully corrected condition (Step S7). This time, the ophthalmologic apparatus 100 maintains the right subject eye in the fully corrected condition and changes the spherical power of the left subject eye from the power in the fully corrected condition by a predetermined power (the addition of +2D) to induce the anisometropia with the addition of +2D only to the right subject eye E (Step S8). Thereby, the ophthalmologic apparatus 100 reproduces a monovision condition in which the right subject eye has been corrected for the far-point vision and the left subject eye has been corrected for the near-point vision.

In the anisometropia condition with the addition of +2D to the left subject eye, the ophthalmologic apparatus 100 of the first embodiment respectively presents the left fixation target 500L to the left subject eye and the right fixation target 500R to the right subject eye while changing the examination distance in a predetermined distance range (0D to 4D). Then, the ophthalmologic apparatus 100 measures the refractive values of the left and right subject eyes E binocularly viewing the fixation targets (the left and right fixation targets 500L, 500R), thereby acquiring the objective measurement information (Step S10, Step S11, Step S12, Step S16, and Step S17, the second step).

In other words, the ophthalmologic apparatus 100 of the first embodiment respectively presents the fixation targets (left and right fixation targets 500L, 500R) to the left and right subject eyes E in the anisometropia condition with the addition of +2D to the left subject eye at a predetermined far-point vision position (examination distance = 0D), and changes the presentation positions of the fixation targets (left and right fixation targets 500L, 500R) in the near-point vision direction while presenting the fixation targets (left and right fixation targets 500L, 500R). Then, the ophthalmologic apparatus 100 measures the refractive values of the subject eyes E viewing the fixation targets (left and right fixation targets 500L, 500R). In the first embodiment, the ophthalmologic apparatus 100 temporarily suspends the change of the examination distance every time when +1D is added to the examination distance (every time when the examination distance reaches 0D, 1D, 2D, 3D, and 4D) until the examination distance changes from 0D to 4D and acquires the refractive values of the left and right subject eyes E, respectively. Further, in the first embodiment, the examiner checks how the left and right fixation targets 500L, 500R are viewed (subjective test) every time when the examination distance increases by a predetermined subjective examination distance (+1D) (every time when the examination distance reaches 0D, 1D, 2D, 3D, and 4D) until the examination distance changes from 0D to 4D (Step S9).

Thus, the ophthalmologic apparatus 100 measures the amount of the adjustment reaction of the left and right subject eyes E even in the anisometropia condition with the addition of +2D only to the left subject eye, from the fully corrected condition of both eyes and checks the fixation targets are viewed at predetermined examination distances (0D, 1D, 2D, 3D and 4D).

Furthermore, after measuring the refractive values, the ophthalmologic apparatus 100 of the first embodiment determines the fixation eye (Step S13), based on the measured refractive values, when the examination distance reaches predetermined subjective examination distances (0D, 1D, 2D, 3D and 4D) in the anisometropia condition with the addition of +2D to the left subject eye. Based on the fixation eye determination result, the ophthalmologic apparatus 100 determines whether or not the fixation eye has switched (Step S14) while the examination distance changes in the anisometropia condition with the addition of +2D to the left subject eye. The display 131 suitably displays the fixation eye determination result and the result of determining whether or not the fixation eye has switched (fixation switching determination).

When the ophthalmologic apparatus 100 has measured the amount of the adjustment reaction in the anisometropia condition with +2D (the addition of +2D) alternately added to the right or left subject eye E (if determined YES in Step S15), the ophthalmologic apparatus 100 finishes the examination of the subject eyes E shown in FIG. 4.

In this way, when inducing the anisometropia in which one of the left and right subject eyes E is maintained in the fully corrected condition and the other of the left and right subject eyes has been corrected from the fully corrected condition by a predetermined power, the visual target projection system 4 in the ophthalmologic apparatus 100 presents the fixation targets (left and right fixation targets 500L, 500R) while changing the examination distance from the left and right subject eyes E to the fixation targets (left and right fixation targets 500L, 500R) in a predetermined distance range (0D to 4D). The controller 140 controls the measurement of the refractive values of both eyes binocularly viewing the fixation targets (left and right fixation targets 500L, 500R) with the refraction measurement projection system 6 and the refraction measurement light receiving system 7, thereby respectively acquiring the refractive values of the left and right subject eyes E as the objective measurement information.

The refraction value of the fixation eye and the refraction value of the non-fixation eye differ from each other in the amount of dissociation and variation relative to the reference refractive value defined for each presentation position of the fixation target. Thus, by acquiring the refractive values of the left and right subject eyes E while changing the examination distance in the ophthalmologic apparatus 100 of the first embodiment, the examiner can identify the fixation eye at every examination distance in the anisometropia condition. Then, the examiner can evaluate a relationship between the subjective viewing of the fixation target and the refractive value (adjustment condition) by using the ophthalmologic apparatus 100 at every predetermined examination distance (0D, 1D, 2D, 3D, 4D) when the monovision condition has been reproduced.

Also, according to the ophthalmologic apparatus 100 of the first embodiment, the examiner can check whether or not the examinee's adjustment ability in the near-point vision decreases by reproducing the monovision condition. Furthermore, the examiner can evaluate the applicability of the examinee to the monovision based on the difference in the adjustment reaction amount and whether the examinee's adjustment ability under the near-point vision decreases or not.

According to the ophthalmologic apparatus 100 of the first embodiment, the examiner can identify the fixation eye at every examination distance under the anisometropia condition and conduct monovision training during the examination. The monovision training allows the examinee to feel the sensation of being able to see a wide range from distance to near without an adjustment by alternating the fixed eyes, for example, by encouraging the examinee to see the fixation target or distance with the subject eye E that has the greater spherical add power (ADD power).

Furthermore, the ophthalmologic apparatus 100 of the first embodiment includes the anterior ocular segment observation system 5 and respectively presents the fixation targets (left and right fixation targets 500L, 500R) to the left and right subject eyes E to acquire the anterior ocular segment image in a condition of binocular fusion. Thereby, it is possible to measure the eye positions (visual line directions) of the left and right subject eyes E. To detect the left visual line direction as the reference of the left subject eye examination and the right visual line direction as the reference of the right subject eye examination, the controller 141 first determines the two-dimensional positions of the pupil center positions of the left and right subject eyes based on the anterior ocular segment images and magnifications of the left and right subject eyes. Next, the main controller 141 determines the reference positions as the two-dimensional positions of the bright spots (bright spot images Br) drawn by the XY-alignment system 2 based on the anterior ocular segment images and magnifications of the left and right subject eyes. Then, the main controller 141 determines the left and right visual line directions based on the reference positions and the pupil center positions. The method for determining the left and right visual lines is not limited to the above method, and other known methods may be used.

When the left and right visual lines are detected, if the difference between the convergence distance calculated from the convergence angle obtained by the detected visual lines and the convergence distance set by controlling the driver 122a is large, or if the examinee complains of a double vision of the fusion targets, it may be possible that the visual targets are not binocularly viewed (not fused). Accordingly, the examiner or main controller 141 may encourage the examinee to direct his or her visual lines to the visual targets to detect the left and right visual lines again.

The main controller 141 of the ophthalmologic apparatus 100 in the first embodiment may temporarily suspend the change of the examination distance and intermittently acquire the objective measurement information (refractive values) when the examination distance has reached a predetermined distance (e.g., 0D, 1D, 2D, 3D, or 4D). In this case, the ophthalmologic apparatus 100 of the first embodiment can reduce the load for calculating the refractive values.

Furthermore, the main controller 141 of the ophthalmologic apparatus 100 in the first embodiment may continuously acquire the objective measurement information (refractive values) while the examination distance is changed in a predetermined range (e.g., 0D to 4D). In this case, the ophthalmologic apparatus 100 can allow the examiner to continuously monitor the fluctuation of the refractive value of the subject eye E with the change of the examination distance. Thereby, the examiner can find the change of the refractive value in detail. This makes it possible to more precisely find the timing of the fixation switching. The ophthalmologic apparatus 100 may display in real-time which eye has been automatically determined as the fixation eye. Thereby, the ophthalmologic apparatus 100 makes it possible to more easily find the timing of the fixation switching.

The ophthalmologic apparatus 100 of the first embodiment includes the visual target projection system 4 of the left measurement optical system 125L (left-eye projection optical system) and the visual target projection system 4 of the right measurement optical system 125R (right-eye projection optical system) controlled by the controller 140. The visual target projection system 4 of the left measurement optical system 125L presents the left fixation target 500L to the left subject eye. The visual target projection system 4 of the right measurement optical system 125R presents the right fixation target 500R to the right subject eye. The controller 140 respectively moves the focusing lenses 45 of the visual target projection systems 4 in the left and right measurement optical systems 125L, 125R in their optical axis directions to adjust the spherical powers of the left and right subject eyes E, thereby inducing an anisometropia.

Thus, the ophthalmologic apparatus 100 of the first embodiment can integrally have the correction function to adjust the spherical powers of the subject eyes E, the visual target presentation function to separately present the fixation targets to the left and right subject eyes E, and the objective measurement function to objectively measure eye characteristics of the subject eyes E.

The ophthalmologic apparatus 100 of the first embodiment, the left fixation target 500L includes the fusion target 502L and the examination target 503L. The fusion target 502L is depicted in a manner that allows binocular fusion while the left and right subject eyes respectively view the left and right fixation targets 500L, 500R. The examination target 503L is depicted not in a manner that allows binocular fusion while the left and right subject eyes respectively view the left and right fixation targets 500L, 500R. The right fixation target 500R includes the fusion target 502R and the examination target 503R. The fusion target 502R is depicted in a manner that allows binocular fusion while the left and right subject eyes respectively view the left and right fixation targets 500L, 500R. The examination target 503R is depicted not in a manner that allows binocular fusion the left and right subject eyes respectively view the left and right fixation targets 500L, 500R.

Thus, the ophthalmologic apparatus 100 of the first embodiment can conduct a subjective examination to identify the fixation eye by asking how the examinee views the examination targets 503L, 503R. In other words, the examiner can determine the fixation eye based on the subjective examination.

In the ophthalmologic apparatus 100 of the first embodiment, the controller 140 includes the fixation eye determiner 145 that determines the fixation eye based on the objective measurement information (refractive values).

Thus, the ophthalmologic apparatus 100 can mechanically determine the fixation eye at every examination distance, and the examiner can easily learn the fixation eye at every examination distance in the anisometropia condition.

Furthermore, in the ophthalmologic apparatus 100 of the first embodiment, the fixation eye determiner 145 determines whether the subject eye E can be the fixation eye by using the following fixation eye conditions (1) and (2). (1) The subject eye with a difference in refractive value that is equal to or lower than a predetermined threshold, in which the difference is measured between a refractive value of the subject eye itself and a refractive value at the presentation position of the fixation target when the fixation target is presented. (2) The subject eye E, among the left and right subject eyes E, whose refractive value more closely matches a refractive value at the presentation position of the fixation target when the examination distance from the fixation target to the subject eye E has changed from the far-point vision to the near-point vision (the subject eye E, among the left and right subject eyes E, that causes a larger decrease in the difference between the refractive value of the subject eye itself and the refractive value at the presentation position of the fixation target when the examination distance has changed from the far-point vision to the near-point vision).

Thus, the ophthalmologic apparatus 100 of the first embodiment can make the determination precisely when mechanically determining the fixation eye.

The examination method of the first embodiment includes a first step and a second step. In the first step, the left subject eye is maintained in the fully corrected condition, an anisometropia is induced with the addition of +2D to the right subject eye, the fixation targets are presented, the examination distance is changed in a predetermined distance range (0D to 4D), and the refractive values of the left and right subject eyes E are acquired as the objective measurement information. In the second step, after acquiring the objective measurement information in the first step, the right subject eye is maintained in the fully corrected condition, an anisometropia is induced with the addition of +2D to the left subject eye, the fixation targets are presented, the examination distance is changed in the predetermined distance range (0D to 4D), and the refractive values of the left and right subject eyes E are acquired as the objective measurement information.

Thus, the examination method of the first embodiment allows the examiner to separately recognize the fixation eye at every examination distance in the anisometropia condition with the addition of +2D to the right subject eye, and the fixation eye at every examination distance in the anisometropia condition with the addition of +2D to the left subject eye. Thereby, the examiner can precisely evaluate the monovision applicability to the examinee.

The examination method of the first embodiment further includes the third step. In the third step, before acquiring the objective measurement information (refractive values) in the anisometropia condition in the first step, a prescription is implemented to make both of the left and right subject eyes into the fully corrected condition, the fixation targets are presented, the examination distance is changed in the predetermined distance range (0D to 4D), and the refractive values of the left and right subject eyes E are acquired as the objective measurement information.

Thus, the examination method of the first embodiment makes it possible to measure the refractive values of the left and right subject eyes E at every examination distance in the fully corrected condition of both eyes and in the anisometropia conditions. Further, the examination method allows the examiner to evaluate the difference of the amount of the adjustment reaction of the subject eyes E between the fully corrected condition of both eyes and the monovision condition. Moreover, the examination method of the first embodiment makes it possible to determine if the fixation eye switching is caused by insufficient capability in the amount of the adjustment reaction owned by the eye or if the fixation eye switching occurs even with sufficient adjustment capability of the eye.

The relationship between the examination distance (0D to 4D) and the refractive value in each of the binocular fully corrected condition, the anisometropia condition with the addition of +2D to the right eye, and the anisometropia condition with the addition of +2D to the left eye for first, second, and third examinees is described with reference to FIGS. 6A to 8C. In each of the first to third examinees, the right subject eye is the dominant eye. The "dominant eye" is the eye in which the visual information transmitted to the brain is predominantly recognized when binocularly viewed. The result of the refractive value measured at a time when the visual target position has changed is plotted to mark a point in FIGS. 6A to 8C, and the resulting adjacent two points are connected by a straight line. However, the refractive value may be continuously measured while changing the visual target position. In this case, the results of the refractive value are shown in a continuous form. Furthermore, the refractive values may be measured multiple times at a time when the visual target position has been changed to a certain position and the multiple results may be averaged to calculate the refractive value at a certain position.

FIGS. 6A to 6C show the results of the first examinee. As shown in FIG. 6A, in the first examinee, when both of the left and right subject eyes E were in the fully corrected condition, the differences of the refractive value of the left subject eye shown by a dotted line and the refractive value of the right subject eye shown by a solid line from the refractive value according to the presentation position of the fixation targets (left and right fixation targets 500L, 500R) shown by a chain line respectively became a predetermined threshold (e.g., 0.5D) or lower regardless of the examination distance. Accordingly, the left and right eyes in the first examinee are determined to be in fixation regardless of the examination distance when both the left and right eyes are in the fully corrected condition.

In contrast with the above, in the first examinee, in the anisometropia condition with the addition of +2D to the right subject eye as shown in FIG. 6B, the difference between the refractive value of the left subject eye itself and the refractive value at the presentation position of the fixation target became roughly in a range from 0D to 0.5 D (threshold or lower), and the difference between the refractive value of the right subject eye itself and the refractive value at the presentation position of the fixation target became roughly in a range from 1.8D to 2D (higher than threshold) regardless of the examination distance. In the subjective examination, in the anisometropia condition with the addition of +2D to the right subject eye, the first examinee answered that the examination target 503L of the left fixation target 500L was seen more clearly regardless of the examination distance. Accordingly, in the anisometropia condition with the addition of +2D to the right subject eye, the left subject eye of the first examinee is determined as the fixation eye regardless of the examination distance and it is determined that there is no fixation switching.

As shown in FIG. 6C, in the first examinee, in the anisometropia condition with the addition of +2D to the left subject eye, the difference between the refractive value of the right subject eye itself and the refractive value at the presentation position of the fixation target became roughly in a range from 0D to 0.4D (lower than threshold), and the difference between the refractive value of the left subject eye itself and the refractive value at the presentation position of the fixation target became roughly in a range from 1.5D to 2D (higher than threshold) regardless of the examination distance. In the subjective examination, in the anisometropia condition with the addition of +2D to the left subject eye, the first examinee answered that the examination target 503R of the right fixation target 500R was seen more clearly regardless of the examination distance. Accordingly, in the anisometropia condition with the addition of +2D to the left subject eye, the fixation eye of the first examinee is determined to be the right subject eye regardless of the examination distance and it is determined that there is no fixation switching.

As a result, it is observed that the first examinee exhibits a tendency to hardly change the fixation eye between the far-point vision and the near-point vision and to see an object by adjusting according to the change in the examination distance. Therefore, the examiner can evaluate that the applicability of monovision to the first examinee is low.

FIGS. 7A to 7C show the results of the second examinee. As shown in FIG. 7A, in the second examinee, when both of the left and right subject eyes E were in the fully corrected condition, and when the examination distance was from 0D to 3D, the difference between the refractive value of the left subject eye itself and the refractive value at the presentation position of the fixation target and the difference between the refractive value of the right subject eye itself and the refractive value at the presentation position of the fixation target became a predetermined threshold (e.g., 0.5D) or lower. When the examination distance was 4D, the difference of the refractive value of each of the left and right subject eyes from the refractive value according to the presentation position of the fixation targets became about 1.2D (higher than the threshold). Accordingly, it is possible to assume that, when both the left and right eyes are in the fully corrected condition and when the examination distance is 4D, the eyes of the second examinee are not in focus due to the adjustment amount being insufficient.

In contrast with the above, in the second examinee, in the anisometropia condition with the addition of +2D to the right subject eye as shown in FIG. 7B, when the examination distance was from 0D to 3D, the difference between the refractive value of the left subject eye itself and the refractive value at the presentation position of the fixation target became roughly 0D (lower than the threshold), and the difference between the refractive value of the right subject eye itself and the refractive value at the presentation position of the fixation target became roughly in a range from 1.5D to 2D (higher than the threshold). On the other hand, when the examination distance was 4D, the difference between the refractive value of the left subject eye itself and the refractive value at the presentation position of the fixation target became roughly 1.2D (higher than the threshold), and the difference between the refractive value of the right subject eye itself and the refractive value at the presentation position of the fixation target became roughly 0.8D (higher than the threshold). Accordingly, in the second examinee, in the anisometropia condition with the addition of +2D to the right subject eye, when the examination distance changes from 3D to 4D, the difference between the refractive value of the left subject eye itself and the refractive value at the presentation position of the fixation target becomes large (from 0.2D to 1.2D) and the difference between the refractive value of the right subject eye itself and the refractive value at the presentation position of the fixation target becomes small (from 1.5D to 0.8D). In other words, when the examination distance changes from 3D to 4D, the refractive value of the right subject eye gets closer to the refractive value at the presentation position of the fixation target. Accordingly, in the anisometropia condition with the addition of +2D to the right subject eye, the left subject eye of the second examinee is determined as the fixation eye when the examination distance is from 0D to 3D and the right subject eye of the second examinee is determined as the fixation eye when the examination distance is 4D. In the subjective examination, in the anisometropia condition with the addition of +2D to the right subject eye, the second examinee answered that the examination target 503R of the right fixation target 500R was seen slightly darker (was seen more easily) than the examination target 503L when the examination distance was 4D. Accordingly, it is determined that the fixation switching has occurred in the second examinee at the examination distance of 4D in the anisometropia condition with the addition of +2D to the right subject eye from both results of the refractive measurement and the subjective examination. From the result of the fixation switching determination, the examiner can confirm that the result of the refractive value measurement and the result of the subjective examination coincide with each other.

As shown in FIG. 7C, in the second examinee, in the anisometropia condition with the addition of +2D to the left subject eye, when the examination distance was from 0D to 3D, the difference between the refractive value of the right subject eye itself and the refractive value at the presentation position of the fixation target became roughly 0D (lower than the threshold), and the difference between the refractive value of the left subject eye itself and the refractive value at the presentation position of the fixation target became roughly 2D (higher than the threshold). On the other hand, when the examination distance was 4D, the difference between the refractive value of the right subject eye itself and the refractive value at the presentation position of the fixation target became roughly 1.2D (higher than the threshold), and the difference between the refractive value of the left subject eye itself and the refractive value at the presentation position of the fixation target became roughly 0.8D (higher than the threshold). Accordingly, in the second examinee, in the anisometropia condition with the addition of +2D to the left subject eye, when the examination distance changes from 3D to 4D, the difference between the refractive value of the right subject eye itself and the refractive value at the presentation position of the fixation target becomes large (from 0D to 1.2D), and the difference between the refractive value of the left subject eye itself and the refractive value at the presentation position of the fixation target becomes small (from 2D to 0.8D). In other words, when the examination distance changes from 3D to 4D, the refractive value of the left subject eye gets closer to the refractive value at the presentation position of the fixation target. Accordingly, in the anisometropia condition with the addition of +2D to the left subject eye, the right subject eye is determined as the fixation eye when the examination distance is from 0D to 3D, and the left subject eye is determined as the fixation eye when the examination distance is 4D. In the subjective examination, in the anisometropia condition with the addition of +2D to the left subject eye, the second examinee answered that the examination target 503L of the left fixation target 500L was seen slightly darker (was seen more easily) than the examination target 503R. Accordingly, it is determined that the fixation switching has occurred in the second examinee at an examination distance of 4D in the anisometropia condition with the addition of +2D to the left subject eye from both results of the refractive measurement and the subjective examination. From the result of the fixation switching determination, the examiner can confirm that the result of the refractive value measurement and the result of the subjective examination coincide with each other.

As a result, it is observed that the second examinee exhibits a tendency to change the fixation eye between the far-point vision and the near-point vision and see an object in the near-point vision by using the eye with the addition of +2D. Accordingly, the examiner can evaluate that the second examinee is a type of high monovision applicability, regardless of which of the left and right subject eyes E is set for the far-point vision or the near-point vision.

FIGS. 8A to 8C show the results of the third examinee. As shown in FIG. 8A, in the third examinee, when both of the left and right subject eyes E are in the fully corrected condition, the difference of the refractive value of each of the left and right subject eyes from the refractive value according to the presentation position of the fixation targets became a predetermined threshold (e.g., 0.5D) or lower regardless of the examination distance. Accordingly, the left and right eyes of the third examinee are also determined to be in fixation regardless of the examination distance when both eyes are in the fully corrected condition.

In contrast with the above, in the third examinee, in the anisometropia condition with the addition of +2D to the right subject eye as shown in FIG. 8B, when the examination distance was 0D and 1D, the difference between the refractive value of the left subject eye itself and the refractive value at the presentation position of the fixation target became roughly 0D (lower than the threshold), and the difference between the refractive value of the right subject eye itself and the refractive value at the presentation position of the fixation target became roughly in a range from 1.5D to 1.8D (higher than the threshold). On the other hand, when the examination distance was in 2D to 4D, the difference between the refractive value of the left subject eye itself and the refractive value at the presentation position of the fixation target became roughly 1.2D to 1.5D (higher than the threshold), and the difference between the refractive value of the right subject eye itself and the refractive value at the presentation position of the fixation target became roughly 0D to 0.2D (lower than the threshold). Accordingly, in the anisometropia condition with the addition of +2D to the right subject eye, the left subject eye of the third examinee is determined as the fixation eye when the examination distance is 0D to 1D and the right subject eye of the third examinee is determined as the fixation eye when the examination distance is 2D to 4D. In the subjective examination, in the anisometropia condition with the addition of +2D to the right subject eye, the third examinee answered that the examination target 503R of the right fixation target 500R was seen darker (was seen more easily) than the examination target 503L when the examination distance was 2D or after. Accordingly, it is determined that the fixation switching has occurred in the third examinee at the examination distance of 2D in the anisometropia condition with the addition of +2D to the right subject eye.

As shown in FIG. 8C, in the third examinee, in the anisometropia condition with the addition of +2D to the left subject eye regardless of the examination distance, the difference between the refractive value of the right subject eye itself and the refractive value at the presentation position of the fixation target became roughly 0.5D (threshold or lower), and the difference between the refractive value of the left subject eye itself and the refractive value at the presentation position of the fixation target became roughly in a range from 1.2D to 2D (higher than threshold value) regardless of the examination distance. In the subjective examination, in the anisometropia condition with the addition of +2D to the left subject eye, the third examinee answered that the examination target 503R of the right fixation target 500R was seen darker (was seen more easily) than the examination target 503L. Accordingly, in the anisometropia condition with the addition of +2D to the left subject eye, the right subject eye of the third examinee is determined as the fixation eye regardless of the examination distance and it is determined that there is no fixation switching.

As a result, it is observed that the third examinee exhibits a tendency to see an object basically with the right subject eye which is the dominant eye. Accordingly, the examiner can evaluate that the third examinee is a type of high monovision applicability where the left subject eye is set for the far-point vision and the right subject eye is set for the near-point vision.

Described above is the first embodiment of the present invention regarding the ophthalmologic apparatus and the method for examining the subject eyes. The specific configurations are not limited to the first embodiment. Design changes, additions, and modifications should be allowed as long as they do not deviate from the gist of the inventions recited in the claims.

The ophthalmologic apparatus 100 of the first embodiment has been described that includes the visual target presenting function, the phoropter function, and the automatic refraction and keratometry measurement function. The configuration of the ophthalmologic apparatus 100 is not limited to that of the first embodiment. For example, the ophthalmologic apparatus 100 may consist of an external visual target device, which is installed on the outside of the apparatus, and a device including a combination of a phoropter function and an automatic refraction and keratometry measurement function. In this case, the visual target presenting position, that is, the examination distance may be manually changed by the examiner or may be changed through a communication control by the controller 140.

The ophthalmologic apparatus 100 of the first embodiment has been described that is configured to change the examination distance and the spherical power (corrected power) of each of the left and right subject eyes E by moving the focusing lens 45 of the visual target projection system 4 in the optical axis direction. The configuration to change the examination distance and the spherical power is not limited to the above. For example, the ophthalmologic apparatus 100 may change the examination distance and the spherical power by moving the display 41 which displays the visual target in the direction of the optical axis. Furthermore, the spherical power of the subject eye E may be changed by suitably placing and removing a plurality of corrective lenses, which is provided in the optical axis direction of the visual target projection system 4 with a configuration to be placed and removed.

The ophthalmologic apparatus 100 of the first embodiment has been described that includes the left fixation target 500L and right fixation target 500R, which configured to be presented as the fixation target to the left subject eye and the right subject eye, respectively. Here, the left and right fixation targets 500L, 500R respectively include the fusion targets 502L, 502R which can be in the condition of the binocular fusion together, and the examination targets 503L, 503R which cannot be in the condition of the binocular fusion together. However, any configuration may satisfy in which the fixation target includes at least the fusion target. Accordingly, the left and right fixation targets 500L, 500R may not include the examination targets 503L, 503R.

The fixation target, which includes the fusion target and the examination target, may be provided as one single target for the left and right subject eyes E. In this case, for example, the examinee may use polarized glasses, etc., and/or a compound-eye-type 3D display to make the left and right subject eyes E individually see the examination target.

The ophthalmologic apparatus 100 of the first embodiment has been described that is configured to change the position of the focusing lens 45 until confirming the fully corrected condition of the left and right subject eyes E by using the RG examination. However, prescribing the fully corrected values for the left and right subject eyes E may be provided, for example, with the use of the corrected values measured by a procedure other than the examination method shown in FIG. 4, with the use of the current eyeglass prescription values already prescribed for the subject eyes E, or with multiple subjective examinations.

The examination method of the first embodiment has been described that measures the refractive values of the left and right subject eyes E at each examination distance even when the left and right subject eyes E are in the fully corrected condition. However, the refractive values under the fully corrected condition may not be measured.

Step S10 of the first embodiment has been described that the main controller 141 continuously changes the presentation position of the fixation target while presenting the fixation target. Changing the presentation position of the fixation targets is not limited to the above. The main controller 141 may change the presentation position of the fixation targets in a stepwise manner by a predetermined distance (e.g., +1D). In this case, the examiner may ask the examinee questions of how the examination targets 503L, 503R are seen every time when the presentation position of the fixation targets is changed. Furthermore, when changing the position of the visual targets in a stepwise manner, the main controller 141 may conceal the visual targets not to be seen by the examinee while moving the visual targets and then present the fixation targets after changing the presentation position of the fixation targets.

In addition, concerning the above description of the first embodiment, the following will be further disclosed.
(1) An ophthalmologic apparatus including:
   a visual target presenting portion that is configured to present fixation targets to left and right subject eyes, respectively;
   an objective measurement optical system that is configured to objectively measure eye characteristics of the left and right subject eyes, respectively; and
   a controller that is configured to control the objective measurement optical system,
   wherein each of the fixation targets includes a fusion target that is depicted in a manner that allows binocular fusion while the left and right subject eyes respectively view the fixation targets,
   wherein the visual target presenting portion is configured to present the fixation targets while changing an examination distance from the left and right subject eyes to the fixation targets in a predetermined range of the examination distance when an anisometropia is induced, in which one of the left and right subject eyes is maintained in a fully corrected condition and another of the left and right subject eyes is corrected by a predetermined degree from the fully corrected condition, and
   wherein the controller is configured to control the objective measurement optical system to measure the eye characteristics while the left and right subject eyes binocularly view the fixation targets and acquire refractive values of the left and right subject eyes as objective measurement information.
(2) The ophthalmologic apparatus according to the above (1), wherein the controller is configured to intermittently acquire the objective measurement information at a time when the examination distance has reached a predetermined distance.
(3) The ophthalmologic apparatus according the above (1), wherein the controller is configured to continuously acquire the objective measurement information while the examination distance is changed in the predetermined range.
(4) The ophthalmologic apparatus according to any one of the above (1) to (3), wherein the controller is configured to control the visual target presenting portion,
   wherein the visual target presenting portion includes
      a left-eye projection optical system that is configured to present a left fixation target of the fixation targets to the left subject eye, and
      a right-eye projection optical system that is configured to present a right fixation target of the fixation targets to the right subject eye, and
   wherein the controller is further configured to control the left-eye and right-eye projection optical systems to induce the anisometropia.
(5) The ophthalmologic apparatus according to the above (4), wherein the left and right fixation targets respectively include
   the fusion targets that are depicted in a manner that allows binocular fusion while the left subject eye views the left fixation target and the right subject eye views the right fixation target, and
   examination targets that are depicted not in a manner that allows binocular fusion while the left subject eye views the left fixation target and the right subject eye views the right fixation target.
(6) The ophthalmologic apparatus according to any one of the above (1) to (5), wherein the controller includes a fixation eye determiner that is configured to determine a fixation eye based on the objective measurement information.
(7) The ophthalmologic apparatus according to the above (6), wherein the fixation eye determiner is configured
   to determine a subject eye among the left and right subject eyes as the fixation eye when the subject eye with a difference in a refractive value that is equal to or less than a predetermined threshold, the difference being measured between a refractive value of the subject eye itself and a refractive value at a presentation position of the fixation target when the fixation target is presented or
   to determine a subject eye among the left and right subject eyes as the fixation eye when the subject eye causes a larger decrease in a difference between the refractive value of the subject eye itself and the refractive value at a presentation position of the fixation target when the examination distance is changed from far-point vision to near-point vision.
(8) A method for examining subject eyes by an ophthalmologic apparatus,
   wherein the ophthalmologic apparatus includes
   a visual target presenting portion that is configured to present fixation targets to left and right subject eyes, respectively;
   an objective measurement optical system that is configured to objectively measure eye characteristics of the left and right subject eyes, respectively; and
   a controller that is configured to control the objective measurement optical system,
   wherein the method includes
   a first step wherein under a condition of an anisometropia in which one of the left and right subject eyes is maintained in a fully corrected condition and another of the left and right subject eyes is corrected by a predetermined power from the fully corrected condition, the visual target presenting portion presents the fixation targets and changes an examination distance from the left and right subject eyes to the fixation target in a predetermined range of the examination distance, and the controller controls the objective measurement optical system to measure the eye characteristics while the left and right subject eyes binocularly view the fixation targets and acquire refractive values of the left and right subject eyes as objective measurement information; and
   a second step wherein after acquiring the objective measurement information in the first step, under a condition of an anisometropia in which the another of the left and right subject eyes is maintained in the fully corrected condition and in which the one of the left and right subject eyes is corrected by a predetermined power from the fully corrected condition, the visual target presenting portion presents the fixation targets and changes the examination distance in the predetermined range of the examination distance, and the controller controls the objective measurement optical system to measure the eye characteristics while both the left and right subject eyes binocularly view the fixation targets and acquire refractive values of the left and right subject eyes as the objective measurement information.
(9) The method according to the above (8), further comprising a third step wherein before acquiring the objective measurement information in the first step, the visual target presenting portion presents the fixation targets and changes the examination distance in the predetermined range of the examination distance while the left and right subject eyes are in the fully corrected condition, and the controller controls the objective measurement optical system to measure the eye characteristics while the left and right subject eyes binocularly view the fixation target and acquire refractive values of the left and right subject eyes as the objective measurement information.

### List of Reference Signs

- 4: visual target projection system (visual target presenting portion)
- 6: refraction measurement projection system (objective measurement optical system)
- 7: refraction measurement light receiving system (objective measurement optical system)
- 100: ophthalmologic apparatus
- 125L: left measurement optical system
- 125R: right measurement optical system
- 122a: driver
- 140: controller
- 141: main controller
- 500L: left fixation target
- 500R: right fixation target
- 502L: fusion target
- 502R: fusion target
- 503L: test target
- 503R: test target

## Claims

1. An ophthalmologic apparatus (100) comprising:
a visual target presenting portion (4) that is configured to present fixation targets (500L, 500R) to left and right subject eyes (E), respectively;
an objective measurement optical system (6, 7) that is configured to objectively measure eye characteristics of the left and right subject eyes (E), respectively; and
a controller (140) that is configured to control the objective measurement optical system (6, 7),
wherein each of the fixation targets (500L, 500R) comprises a fusion target (502L, 502R) that is depicted in a manner that allows binocular fusion while the left and right subject eyes (E) respectively view the fixation targets (500L, 500R),
wherein the visual target presenting portion (4) is configured to present the fixation targets (500L, 500R) while changing an examination distance from the left and right subject eyes (E) to the fixation targets (500L, 500R) in a predetermined range of the examination distance when an anisometropia is induced, in which one of the left and right subject eyes (E) is maintained in a fully corrected condition and another of the left and right subject eyes (E) is corrected by a predetermined degree from the fully corrected condition, and
wherein the controller (140) is configured to control the objective measurement optical system (6, 7) to measure the eye characteristics while the left and right subject eyes (E) binocularly view the fixation targets (500L, 500R) and acquire refractive values of the left and right subject eyes (E) as objective measurement information.

2. The ophthalmologic apparatus (100) according to claim 1, wherein the controller (140) is configured to intermittently acquire the objective measurement information at a time when the examination distance has reached a predetermined distance.

3. The ophthalmologic apparatus (100) according to claim 1, wherein the controller (140) is configured to continuously acquire the objective measurement information while the examination distance is changed in the predetermined range.

4. The ophthalmologic apparatus (100) according to any one of claims 1 to 3, wherein the controller (140) is configured to control the visual target presenting portion (4),
wherein the visual target presenting portion (4) comprises
a left-eye projection optical system (4) that is configured to present a left fixation target (500L) of the fixation targets (500L, 500R) to the left subject eye (E), and
a right-eye projection optical system (4) that is configured to present a right fixation target (500R) of the fixation targets (500L, 500R) to the right subject eye (E), and
wherein the controller (140) is further configured to control the left-eye and right-eye projection optical systems (4) to induce the anisometropia.

5. The ophthalmologic apparatus (100) according to claim 4, wherein the left and right fixation targets (500L, 500R) respectively comprise
the fusion targets (502L, 502R) that are depicted in a manner that allows binocular fusion while the left subject eye (E) views the left fixation target (500L) and the right subject eye (E) views the right fixation target (500R), and
examination targets that are depicted not in a manner that allows binocular fusion while the left subject eye (E) views the left fixation target (500L) and the right subject eye (E) views the right fixation target (500R).

6. The ophthalmologic apparatus (100) according to any one of claims 1 to 3, wherein the controller (140) comprises a fixation eye determiner (145) that is configured to determine a fixation eye based on the objective measurement information.

7. The ophthalmologic apparatus (100) according to claim 6, wherein the fixation eye determiner is configured
to determine a subject eye (E) among the left and right subject eyes (E) as the fixation eye when the subject eye (E) with a difference in a refractive value that is equal to or less than a predetermined threshold, the difference being measured between a refractive value of the subject eye (E) itself and a refractive value at a presentation position of the fixation target (500L, 500R) when the fixation target (500L, 500R) is presented, or
to determine a subject eye (E) among the left and right subject eyes (E) as the fixation eye when the subject eye (E) causes a larger decrease in a difference between the refractive value of the subject eye (E) itself and the refractive value at a presentation position of the fixation target (500L, 500R) and when the examination distance is changed from far-point vision to near-point vision.

8. A method for examining subject eyes (E) by an ophthalmologic apparatus (100),
wherein the ophthalmologic apparatus (100) comprises:
a visual target presenting portion (4) that is configured to present fixation targets (500L, 500R) to left and right subject eyes (E), respectively;
an objective measurement optical system (6, 7) that is configured to objectively measure eye characteristics of the left and right subject eyes (E), respectively; and
a controller (140) that is configured to control the objective measurement optical system (6, 7),
wherein the method comprises:
a first step wherein under a condition of an anisometropia in which one of the left and right subject eyes (E) is maintained in a fully corrected condition and another of the left and right subject eyes (E) is corrected by a predetermined power from the fully corrected condition, the visual target presenting portion (4) presents the fixation targets (500L, 500R) and changes an examination distance from the left and right subject eyes (E) to the fixation target (500L, 500R) in a predetermined range of the examination distance, and the controller (140) controls the objective measurement optical system (6, 7) to measure the eye characteristics while the left and right subject eyes (E) binocularly view the fixation targets (500L, 500R) and acquire refractive values of the left and right subject eyes (E) as objective measurement information and
a second step wherein after acquiring the objective measurement information in the first step, under a condition of an anisometropia in which the another of the left and right subject eyes (E) is maintained in the fully corrected condition and the one of the left and right subject eyes (E) is corrected by a predetermined power from the fully corrected condition, the visual target presenting portion (4) presents the fixation targets (500L, 500R) and changes the examination distance in the predetermined range of the examination distance, and the controller (140) controls the objective measurement optical system (6, 7) to measure the eye characteristics while the left and right subject eyes (E) binocularly view the fixation targets (500L, 500R) and acquire refractive values of the left and right subject eyes (E) as the objective measurement information.

9. The method according to claim 8, further comprising a third step wherein before acquiring the objective measurement information in the first step, the visual target presenting portion (4) presents the fixation targets (500L, 500R) and changes the examination distance in the predetermined range of the examination distance while the left and right subject eyes (E) are in the fully corrected condition, and the controller (140) controls the objective measurement optical system (6, 7) to measure the eye characteristics while the left and right subject eyes (E) binocularly view the fixation target (500L, 500R) and acquire refractive values of the left and right subject eyes (E) as the objective measurement information.
